# EUROPÄISCHE PATENTSCHRIFT

(11) **EP 3 752 227 B1**
(45) Veröffentlichungstag und Bekanntmachung des Hinweises auf die Patenterteilung: **31.01.2024**
(21) Anmeldenummer: 19710480.5
(22) Anmeldetag: 06.02.2019
(51) Int. Cl.: A61M 5/315, A61M 5/24, A61M 5/31

(54) **VERBINDUNGSMECHANISMUS FÜR ZUSATZMODUL**
CONNECTION MECHANISM FOR ADD-ON MODULE
MÉCANISME DE CONNEXION POUR MODULE D'EXTENSION

(30) Priorität: 15.02.2018 CH 1832018
(43) Veröffentlichungstag der Anmeldung: 23.12.2020
(73) Patentinhaber: Ypsomed AG, 3401 Burgdorf (CH)
(72) Erfinder: URBANEK, Leos, 3012 Bern (CH); HIRSCHEL, Jürg, 3007 Bern (CH); ALLENSPACH, Marcel, 3400 Burgdorf (CH); KALBERMATTER, Gabriel, 3400 Burgdorf (CH)
(74) Vertreter: Meier Obertüfer, Jürg
(86) Internationale Anmeldenummer: PCT/IB2019/050927
(87) Internationale Veröffentlichungsnummer: WO 2019/159035

(56) Entgegenhaltungen:
- WO-A1-2010/037828
- WO-A1-2010/098927
- WO-A1-2013/004843
- WO-A1-2013/120774
- WO-A2-2010/128493

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen wie Insulin- und Hormonpräparationen. Die Erfindung bezieht sich auf einen Verbindungsmechanismus eines Zusatzmoduls für eine lösbare Verbindung des Zusatzmoduls mit einem Injektionsgerät.

### HINTERGRUND DER ERFINDUNG

Aus dem Stand der Technik sind Zusatzmodule bekannt, welche lösbar mit einem Injektor verbunden werden können. Solche Zusatzmodule sind wiederverwendbar und können mit einem Einweginjektor wie beispielsweise einen Autoinjektor oder mit einem Mehrweginjektor verbunden werden. Die Zusatzmodule werden auch als Elektronikmodul, "Smart device", "Smart module" oder "Add-on" bezeichnet. Die Zusatzmodule dienen dazu Vorgänge, Ereignisse und Funktionen des Einweginjektors zu erkennen, zu überwachen und erfasste Ereignisse dem Anwender zu melden. So kann zum Beispiel der Anwender beim Injektionsvorgang in Echtzeit durch das Zusatzmodul unterstützt werden, indem auf einer Applikation auf einem Smartphone Instruktionen für die Injektion, Vorschläge für die Injektionszeit und Handhabungsfehler angezeigt werden. Ferner können mittels des Zusatzmoduls visuelle und akustische Rückmeldung für spezielle Ereignisse wie zum Beispiel das erfolgreiche Abschliessen einer Injektion ausgegeben werden. Das Zusatzmodul kann zudem Informationen über getätigte Injektionen aufzeichnen und weiterleiten oder anhand der aufgezeichneten Daten Auswertungen vornehmen.

Das Zusatzmodul wird vor dem Injektionsbeginn mit einem Injektor verbunden, indem dieses lösbar auf den Einweginjektor aufgesetzt oder aufgesteckt wird. Für diese Verbindung des Zusatzmoduls mit dem Injektor sind verschiedene Verbindungsmechanismen bekannt.

Eine solche lösbare Verbindung zwischen Zusatzmodul und Injektor beschreibt beispielsweise die Patentanmeldung US 2015/0025470 A1. Um den Injektor mit dem Zusatzmodul zu verbinden, wird der Injektor in eine Öffnung des Zusatzmoduls eingeführt. Wenn der Injektor in Rotationsrichtung korrekt zum Zusatzmodul ausgerichtet ist, wird eine auf einer Aussenfläche des Injektors angebrachte Rippe in einer Nut im Zusatzmodul aufgenommen und der Injektor kann vollständig in das Zusatzmodul eingeführt werden. Um das Zusatzmodul am Injektor zu befestigen, dreht der Benutzer an einem Sicherungsring, welcher in Längsrichtung ausgerichtete Sicherungsarme umgreift und mit diesen in Gewindeverbindung steht. Durch die Drehung wird der Sicherungsring in Längsrichtung und relativ zum Injektor bewegt, wodurch die freien Enden der Sicherungsarme radial nach innen auf die Aussenfläche des Injektors gedrückt werden. Das Zusatzmodul wird dadurch am Injektor gehalten.

Diese Verbindung hat den Nachteil, dass der Benutzer mehrere Schritte, nämlich das Aufschieben und das anschliessende Festschrauben mit dem Sicherungsring vornehmen muss, um das Zusatzmodul mit dem Injektor sicher verbinden zu können.

Die WO 2010/128493 A2 offenbart ein Überwachungsmodul für einen Injektionspen, welches eine Hülse und einen Universalkopf umfasst. Der Universalkopf kann mittels Befestigungselementen auf der Hülse angebracht werden. Die Hülse umschliesst dabei ein Gehäuse des Injektionspens. Wenn der Universakopf montiert ist, wird die Hülse komprimiert, so dass sie das Gehäuse des Injektionspens fest umschliesst und damit das Überwachungsmodul fest am Injektionspen hält. In diesem montierten Zustand kann das Überwachungsmodul Injektionsvorgänge mit dem Injektionspen überwachen, aufnehmen und gesammelte Informationen einem Benutzer melden.

Einen weiteren Verbindungsmechanismus zum Befestigen eines Zusatzmoduls an einem Injektor beschreibt die Patentanmeldung WO 2013/120775 A1. Zum Verbinden wird der Injektor in eine Öffnung im Zusatzmodul eingeführt. Dabei muss das Zusatzmodul zum Injektor angewinkelt werden, so dass die Längsachse des Zusatzmoduls in einem spitzen Winkel zur Längsachse des Injektors steht. Anschliessend wird das Zusatzmodul zum Injektor hin verschwenkt, so dass die beiden Längsachsen parallel zueinander ausgerichtet sind. Beim Verschwenken werden zwei seitliche Flügel des Zusatzmoduls auseinandergedrückt. Wenn das Zusatzmodul ganz auf den Injektor heruntergeschwenkt wird, bewegen sich die Flügel wieder etwas gegeneinander zu. Hierbei greift je ein Vorsprung am jeweiligen Ende der Flügel in eine seitliche Ausnehmung im Injektor ein. Da die Flügel zueinander hin vorgespannt sind und somit von beiden Seiten auf den Injektor drücken, werden die Vorsprünge der Flügel in die Ausnehmungen hineingeschnappt.

Auch bei dieser Verbindung kann der Benutzer den Injektor nicht in einem Vorgang mit dem Zusatzmodul verbinden, sondern muss mehrere Schritte vornehmen. Zudem fehlt eine Sicherung oder Verriegelung der Verbindung.

Die WO 2003/011370 A2 offenbart einen Verbindungsmechanismus, der einen Schnapper umfasst, der in eine Nut im Injektor eingreift. Dabei wird der Schnapper zusätzlich mittels einer Druckfeder gegen den Injektor gedrückt. Zu Beginn und während der Ausschüttung sichert ein Verriegelungsschieber, welcher an die Antriebsvorrichtung gekoppelt ist, den Schnapper, so dass sich dieser nicht aus der Nut lösen kann. Am Ende einer Ausschüttung gibt der Verriegelungsschieber den Schnapper frei. Der Injektor kann vom Zusatzmodul gelöst werden.

Bei diesem Verbindungsmechanismus muss der Benutzer zum Verbinden des Injektors mit dem Zusatzmodul während dem Einführen des Injektors den Löseknopf gedrückt halten und gleichzeitig den Injektor in Rotationsrichtung positionieren. Das erschwert die Handhabung für den Benutzer.

### DARSTELLUNG DER ERFINDUNG

Es ist eine Aufgabe der Erfindung, eine einfache und sichere Verbindung von Zusatzmodul und Injektor zu ermöglichen. Diese Aufgabe wird gelöst durch ein Zusatzmodul und ein Verfahren zum Verbinden eines Injektors mit einem Zusatzmodul gemäss den unabhängigen Ansprüchen. Bevorzugte Ausführungsformen sind Gegenstand der abhängigen Ansprüche.

Gemäss der Erfindung umfasst das Zusatzmodul ein Gehäuse mit einer Halteeinrichtung und einer Aufnahmeeinheit mit einem Halteelement, wobei die Aufnahmeeinheit relativ zum Gehäuse bewegbar ist und wobei
a. das Zusatzmodul eine Haltekonfiguration aufweist, in der die Aufnahmeeinheit durch die Halteeinrichtung im Gehäuse gehalten ist und in der das Halteelement derart mit dem Injektor zusammenwirken kann, dass der Injektor durch die Aufnahmeeinheit gehalten ist;
b. das Zusatzmodul eine Freigabekonfiguration aufweist, in der die Aufnahmeeinheit von der Halteeinrichtung freigegeben ist und der Injektor vom Halteelement freigegeben ist.

Dabei ist das Zusatzmodul durch eine Verstellbewegung der Aufnahmeeinheit von der Haltekonfiguration in die Freigabekonfiguration und zurück verstellbar.

Indem durch eine einfache Verstellbewegung der Aufnahmeeinheit das Zusatzmodul von der Haltekonfiguration, in welcher der Injektor im Gehäuse des Zusatzmoduls gehalten ist, in die Freigabekonfiguration, in welcher der Injektor vom Halteelement freigegeben ist, und zurück verstellbar ist, wird das Verbinden des Injektors mit dem Zusatzmodul und das Trennen des Injektors vom Zusatzmodul erleichtert. Das bedeutet, der Benutzer kann mit einer einzigen Bewegung der Aufnahmeeinheit, welche beispielsweise durch Einschieben des Injektors in das Gehäuse erzeugt werden kann, das Zusatzmodul in die Haltekonfiguration verstellen und dadurch auf einfache Art und Weise den Injektor mit dem Zusatzmodul verbinden.

Dabei wird ohne weitere Verfahrensschritte des Benutzers die Aufnahmeeinheit am Ende der Verstellbewegung durch die Halteeinrichtung im Gehäuse gehalten. Zudem wird durch die einzige Verstellbewegung das Halteelement verstellt, so dass dieses den Injektor halten kann.

Ebenso einfach kann der Injektor wieder vom Zusatzmodul freigegeben werden. Nach dem Lösen der Aufnahmeeinheit von der Halteeinrichtung, kann mit einer einzigen Verstellbewegung der Aufnahmeeinheit das Zusatzmodul von seiner Haltekonfiguration in seine Freigabekonfiguration verstellt werden. Während dieser einzigen Verstellbewegung wird das Halteelement vom Injektor gelöst, so dass das Halteelement zurückgezogen werden kann und dadurch den Injektor von der Aufnahmeeinheit freigeben kann. Dabei kann beispielsweise durch ein elastisches Element ein Teil des Mechanismus automatisiert werden, so dass zum Beispiel der Benutzer nur die Aufnahmeeinheit von der Halteeinrichtung lösen muss und die Verstellbewegung durch das Lösen automatisch ausgelöst wird.

Zudem ist durch die erfindungsgemässe Verbindung der Injektor sicher mit dem Zusatzmodul verbunden und das Zusatzmodul kann sich nicht unerwünscht vom Injektor lösen. So bleibt zum Beispiel das Zusatzmodul auch dann sicher mit dem Injektor verbunden, wenn der Benutzer im distalen, einstechseitigen Bereich des Injektors einen Verschluss abzieht und den Injektor über das Zusatzmodul im proximalen Bereich hält.

Unter dem Begriff "Injektor" wird in der vorliegenden Beschreibung eine Vorrichtung verstanden, bei der die Injektionsnadel oder Kanüle unmittelbar oder nach einer Haltezeit kleiner 1 min. nach Abgabe der medizinischen Substanz aus dem Gewebe des Patienten wieder entfernt wird. Somit verbleibt bei einem Injektor im Unterschied zu einem Infusionssystem die Injektionsnadel oder Kanüle nicht permanent bzw. über einen längeren Zeitraum von mehreren Stunden im Patienten.

Beim Injektor kann es sich um einen Einweginjektor oder einen Mehrweginjektor handeln. Ein Einweginjektor ist ein Injektor, welcher dazu verwendet wird, die in einer nicht nachfüllbaren und nicht austauschbaren Karpule oder vorgefüllten Spritze befindliche medizinische Substanz subkutan zu injizieren. Ist die zur Injektion vorgesehene Menge der medizinischen Substanz in einem oder in mehreren Injektionsvorgängen injiziert worden, wird der Einweginjektor entsorgt. Die Karpule oder vorgefüllten Spritze im Einweginjektor kann nicht ausgetauscht werden. Demgegenüber ist bei einem Mehrweginjektor die Karpule oder vorgefüllten Spritze mit der medizinischen Substanz auswechselbar.

Der Injektor weist vorzugsweise eine längliche Form auf, welche eine Längsachse definiert. Die in der vorliegenden Beschreibung verwendeten Begriffe "axial" und "Längsachse" beziehen sich beide auf eine Richtung parallel zu dieser Längsachse des Injektors. Weiter bezieht sich die Angabe "distal" auf eine Richtung zum einstechseitigen Ende des Injektors hin. Demgegenüber bezieht sich die Angabe "proximal" auf eine Richtung zum hinteren, dem einstechseitigen Ende gegenüberliegenden Ende des Injektors hin.

Der Begriff "Medikament" oder "medizinische Substanz" umfasst in diesem Zusammenhang jede fliessfähige medizinische Formulierung, welche geeignet ist zur kontrollierten Verabreichung mittels einer Kanüle oder Hohlnadel, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Die Aufnahmeeinheit weist mindestens ein Halteelement auf zum Halten des Injektors an oder in der Aufnahmeeinheit. Vorzugsweise greift das Halteelement in der Haltekonfiguration in eine Aussparung im Injektor ein, um den Injektor zu halten. In einer bevorzugten Ausführung weist die Aufnahmeeinheit mindestens zwei Halteelemente auf, damit der Injektor zuverlässig gehalten werden kann. Der Einfachheit halber wird in der nachfolgenden Beschreibung für das Halteelement nur die Einzahl verwendet. Falls die Aufnahmeeinheit mehrere Halteelemente aufweist, beziehen sich die erwähnten Merkmale auf alle Halteelemente der Aufnahmeeinheit.

Das Halteelement ist vorzugsweise als Haltearm ausgebildet, welcher relativ zur Aufnahmeeinheit bewegbar ist. In alternativen Ausführungen kann das Halteelement jedoch zum Beispiel auch als Haken ausgebildet sein.

Vorzugsweise ist das Halteelement in der Haltekonfiguration bis auf ein kleines Spiel nicht bewegbar. Dadurch ist der Injektor in der Haltekonfiguration sicher an der Aufnahmeeinheit gehalten.

Die Aufnahmeeinheit dient dazu den Injektor im Gehäuse des Zusatzmoduls in der Haltekonfiguration zu halten. Die Aufnahmeeinheit selber ist mittels der Halteeinrichtung in der Haltekonfiguration im Gehäuse des Zusatzmoduls gehalten. Somit ist der Injektor nicht direkt sondern über die Aufnahmeeinheit im Gehäuse des Zusatzmoduls gehalten. Vorzugsweise ist die Aufnahmeeinheit hülsenförmig ausgebildet und der Injektor ist in die Hülsenform der Aufnahmeeinheit einführbar. Die Aufnahmeeinheit kann jedoch auch eine andere Form aufweisen. Zum Beispiel kann sie als Halbschale oder als Behälter ausgestaltet sein. Zudem kann die Aufnahmeeinheit beispielsweise auch als Platte mit einer umlaufenden Wandung, welche eine Kavität bildet, in die der Injektor aufgenommen werden kann, ausgebildet sein. Unabhängig von der Form der Aufnahmeeinheit ist diese relativ zum Gehäuse bewegbar. Vorzugsweise ist die Aufnahmeeinheit verschiebbar am Gehäuse geführt. Alternativ kann die Aufnahmeeinheit jedoch auch drehbar oder verschieb- und drehbar am Gehäuse gelagert sein.

Das Gehäuse weist vorzugsweise eine Öffnung auf, in der sich die Aufnahmeeinheit befindet. Diese Form des Gehäuses ist jedoch nicht zwingend. Das Gehäuse kann beispielsweise halbschalenförmig oder als rechteckiger oder zylindrischer Körper mit einer einseitigen Öffnung ausgebildet sein. Ferner kann das Gehäuse als Hohlkörper mit einer durchgehenden Öffnung ausgestaltet sein.

In der Haltekonfiguration hält die Halteeinrichtung die Aufnahmeeinheit im Gehäuse, so dass die Aufnahmeeinheit nicht frei relativ zum Gehäuse bewegbar ist. Vorzugsweise ist die Aufnahmeeinheit durch die Halteeinrichtung derart gehalten, dass sie bis auf ein kleines mechanisches Spiel nicht relativ zum Gehäuse bewegbar ist. In der Freigabekonfiguration hingegen ist die Aufnahmeeinheit von der Halteeinrichtung freigegeben. Die Aufnahmeeinheit ist dann vorzugsweise immer noch am Gehäuse gelagert, so dass sie sich nicht vom Gehäuse lösen kann, sie ist jedoch frei relativ zum Gehäuse bewegbar. Vorzugsweise dienen der Halteeinrichtung zum Halten der Aufnahmeeinheit am Gehäuse Haken, Nocken oder Auskragungen. Mit Vorteil kann ein Nocken in ein entsprechend ausgeformtes Gegenelement in der Aufnahmeeinheit eingreifen, um in der Haltekonfiguration die Aufnahmeeinheit im Gehäuse zu halten.

Das Zusatzmodul kann die Haltekonfiguration oder die Freigabekonfiguration einnehmen. Vorzugsweise ist das Zusatzmodul mit einer einzigen Verstellbewegung der Aufnahmeeinheit von der Haltekonfiguration in die Freigabekonfiguration oder von der Freigabekonfiguration in die Haltekonfiguration verstellbar oder überführbar. In der Haltekonfiguration ist der Injektor unbeweglich mittels mindestens eines Halteelements an der Aufnahmeeinheit gehalten, welche wiederum mittels der Halteeinrichtung im Gehäuse des Zusatzmoduls gehalten ist. Vorzugsweise ist der Injektor über die Aufnahmeeinheit derart mit dem Zusatzmodul verbunden, dass das Zusatzmodul mittels Sensoren Aenderungen oder Bewegungen des Injektors, insbesondere Injektionsereignisse im Injektor, erkennen kann. Demgegenüber ist in der Freigabekonfiguration der Injektor nicht in der Aufnahmeeinheit gehalten und somit nicht mit dem Zusatzmodul verbunden. Das bedeutet, in der Freigabekonfiguration ist der Injektor vom Zusatzmodul getrennt und das Zusatzmodul kann Aenderungen oder Bewegungen des Injektors, insbesondere Injektionsereignisse im Injektor, nicht erkennen. In der Freigabekonfiguration kann der Injektor vom Zusatzmodul entfernt werden.

Vorzugsweise ist in der Haltekonfiguration das Halteelement durch das Gehäuse verriegelt. Der Injektor ist also nicht nur im Gehäuse des Zusatzmoduls gehalten, sondern zusätzlich mit einer Verriegelung gegen unerwünschtes Lösen gesichert. Der Begriff "verriegelt" bedeutet, dass sich das Halteelement nicht aus einer Position, in welcher es den Injektor halten kann, wegbewegen kann. Das Halteelement wird also an einer Bewegung in eine Freigaberichtung durch die Verriegelung gehindert. Um den Injektor vom Zusatzmodul zu trennen, muss demnach mindestens die Verriegelung des Halteelements gelöst werden und das Halteelement vom Injektor wegbewegt werden.

Wenn das Halteelement nicht durch das Gehäuse verriegelt ist, kann das Halteelement den Injektor zwar immer noch im Gehäuse halten, das Halteelement ist jedoch aus der Halteposition ohne weiteres wegbewegbar. Für die Verriegelung des Halteelements wirkt das Gehäuse direkt oder indirekt über ein zum Gehäuse gehörendes Zwischenelement mit dem Halteelement zusammen. Vorzugsweise umgibt das Gehäuse oder ein zum Gehäuse gehörendes Zwischenelement das Halteelement zumindest teilweise, um das Halteelement zu verriegeln. Bevorzugt wird am Ende der Verstellbewegung das Halteelement durch das Gehäuse verriegelt.

In einer bevorzugten Ausführung ist die Aufnahmeeinheit durch eine lineare Verstellbewegung verschiebbar zum Verstellen oder zum Überführen des Zusatzmoduls von der Haltekonfiguration in die Freigabekonfiguration und zurück. Das bedeutet, die Verstellbewegung ist keine Dreh- oder Schraubbewegung, sondern eine gradlinige Bewegung zum gradlinigen Verschieben der Aufnahmeeinheit relativ zum Gehäuse. Das bietet den Vorteil, dass die Aufnahmeeinheit besonders rasch verstellt werden kann und dadurch das Zusatzmodul entsprechend rasch von der Haltekonfiguration in die Freigabekonfiguration oder von der Freigabekonfiguration in die Haltekonfiguration verstellbar ist. Die lineare Verstellbewegung erfolgt vorzugsweise parallel zur Längsachse des Gehäuses. Dadurch kann die Verstellbewegung durch eine Relativbewegung zwischen Injektor und Zusatzmodul zum Beispiel durch Einschieben des Injektors in das Zusatzmodul erzeugt werden.

In einer alternativen Ausführung kann die Verstellbewegung der Aufnahmeeinheit zum Beispiel eine Dreh- oder Schraubbewegung sein.

Vorzugsweise ist das Gehäuse hülsenförmig ausgebildet und umgibt den in das Gehäuse eingeführten Injektor. Bevorzugt führt dabei das hülsenförmige Gehäuse den Injektor in einer Einführrichtung. Dabei ist nicht zwingend, dass das Gehäuse in der Haltekonfiguration den Injektor allseitig umgibt. Vorzugsweise umgibt das Gehäuse den Injektor nur in Bezug auf seine Mantelflächen. Das bedeutet, in der Haltekonfiguration ist mindestens eine Stirnfläche des Injektors nicht vom Gehäuse umgeben. Zudem umgibt das Gehäuse den Injektor in Bezug auf die Länge des Injektors in einer bevorzugten Ausführung nur teilweise. Mit Vorteil kann die Aufnahmeeinheit, insbesondere eine hülsenförmige Aufnahmeeinheit, in der Hülsenform des Gehäuses aufgenommen werden.

Mit Vorteil weist die Aufnahmeeinheit ein axiales Anschlagelement für den Injektor auf. Das ermöglicht ein einfaches und rasches Positionieren des Injektors relativ zur Aufnahmeeinheit in axialer Richtung des Gehäuses. Das ist insbesondere dann wichtig, wenn das Halteelement mit einer bestimmten Stelle am Injektor, beispielsweise mit einer Aussparung, zusammenwirken muss, um den Injektor zu halten. Zudem ist die Positionierung des Injektors relativ zum Gehäuse von Bedeutung, wenn der Injektor und das Gehäuse je elektrische Kontakte umfassen, die in der Haltekonfiguration zueinander ausgerichtet sein müssen.

Bevorzugt umfasst das Anschlagelement eine Anschlagfläche, die rechtwinklig zur Längsachse des Gehäuses ausgerichtet ist und auf die eine Stirnseite des Injektors anschlägt, wenn der Injektor in das Gehäuse des Zusatzmoduls eingeführt wird. Mit Vorteil ist die Anschlagfläche zusammen mit der Aufnahmeeinheit relativ zum Gehäuse bewegbar. In diesem Fall kann mittels des Anschlagelements die Aufnahmeeinheit bewegt werden.

Alternativ dazu besteht auch die Möglichkeit, dass die Aufnahmeeinheit kein Anschlagelement aufweist. Der Injektor muss in diesem Fall mittels einer Markierung relativ zum Gehäuse positioniert werden und die Aufnahmeeinheit muss manuell relativ zum Gehäuse verschoben werden.

Vorzugsweise ist die Aufnahmeeinheit durch ein elastisches Element in Richtung einer Längsachse des Gehäuses in der Haltekonfiguration vorgespannt. Bevorzugt wird das elastische Element beim Verstellen des Zusatzmoduls von der Freigabekonfiguration in die Haltekonfiguration gespannt und beim Verstellen von der Haltekonfiguration in die Freigabekonfiguration entspannt. So kann zum Beispiel durch Lösen oder Reduzieren der Vorspannung die Verstellbewegung der Aufnahmeeinheit erzeugt werden, wodurch das Zusatzmodul von seiner Haltekonfiguration in seine Freigabekonfiguration verstellt werden kann. Das elastische Element kann zum Beispiel als mechanische Feder oder als elastisches Kunststoffelement ausgeführt sein.

In einer bevorzugten Ausführung ist das Halteelement zum Halten des Injektors im Wesentlichen in eine Richtung senkrecht zur Längsachse des Zusatzmoduls bewegbar und weist einen Vorsprung zum Zusammenwirken mit dem Injektor auf. Wird das Halteelement in dieser Richtung zum eingesetzten Injektor hin bewegt, gelangt es in eine Halteposition, greift in den Injektor ein und hält diesen an der Aufnahmeeinheit. Wird das Halteelement vom Injektor wegbewegt, wird der Injektor vom Halteelement freigegeben. Das Halteelement ist dabei vorzugsweise mit der Aufnahmeeinheit verbunden. Das bedeutet, das Halteelement wird nicht durch die Aufnahmeeinheit geführt, sondern ist mit dieser vorzugsweise über ein Gelenk bewegbar verbunden.

Der Vorsprung kann die Form einer Nase, eines Nockens, einer Halbkugel oder eines Hakens aufweisen, wobei der Vorsprung in eine entsprechend ausgeformte Aussparung, Ausnehmung, Vertiefung oder Nut im Injektor eingreifen kann. Falls das Halteelement als Haltearm ausgebildet ist, befindet sich der Vorsprung vorzugsweise in einem Bereich eines freien Endes des Haltearms.

Bevorzugt ist das Halteelement schwenkbar und insbesondere elastisch schwenkbar an der Aufnahmeeinheit gehalten. Durch die Schwenkbewegung kann das Halteelement einfach und rasch zum eingesetzten Injektor geschwenkt werden, um diesen zu halten. Falls das Halteelement zudem elastisch schwenkbar ist, kann es gegen den Injektor vorgespannt werden. Wenn der Injektor in das Gehäuse eingeführt wird, schnappt in diesem Fall das Halteelement mit dem Vorsprung in eine Aussparung im Injektor ein und hält dadurch den Injektor zuverlässig. So wird ein einfaches Verbinden des Injektors mit dem Zusatzmodul ermöglicht.

Vorzugsweise umfasst das Halteelement im Bereich eines freien Endes des Halteelements eine Auskragung zum Eingreifen in das Gehäuse zwecks forcierter Lösung des Nockens vom Injektor. Das freie Ende des Halteelements ist hier dasjenige Ende, welches nicht mit der Aufnahmeeinheit verbunden oder durch die Aufnahmeeinheit gehalten oder geführt ist. In einer alternativen Ausführung besteht aber auch die Möglichkeit, dass die Auskragung zwischen den Enden oder in der Mitte des Halteelements angeordnet ist.

In einer bevorzugten Ausführung wird die Auskragung durch das Gehäuse derart geführt, dass der Vorsprung gleichzeitig vom im Gehäuse eingesetzten Injektor zurückgezogen wird, wenn das Halteelement vom eingesetzten Injektor wegbewegt wird. Weiter wird bevorzugt die Auskragung aus der Gehäusewand in Richtung Mitte des Gehäuses herausbewegt und gleichzeitig greift der Vorsprung in den im Gehäuse eingesetzten Injektor ein, wenn das Halteelement zum eingesetzten Injektor hin bewegt wird. Die Auskragung dient somit dazu, das Halteelement geführt vom Injektor weg zu bewegen oder geführt zum Injektor hin zu bewegen.

Das Eingreifen der Auskragung in das Gehäuse ist insbesondere vorteilhaft, wenn das Halteelement zum in das Gehäuse eingesetzten Injektor hin elastisch vorgespannt ist. In diesem Fall muss nämlich das Halteelement zum Lösen vom Injektor gegen die Vorspannkraft vom Injektor wegbewegt werden. Dies kann erreicht werden, indem die Auskragung in das Gehäuse eingeführt wird und dadurch das Halteelement vom eingesetzten Injektor geführt vom Injektor wegbewegt wird.

In einer bevorzugten Ausführung ist das Halteelement als länglicher Haltearm ausgebildet, wobei sich die Auskragung an einer ersten Seite des freien Endes des Haltearms befindet und sich der Vorsprung auf einer der ersten Seite gegenüberliegenden zweiten Seite des freien Endes des Haltearms befindet.

In einer alternativen Ausführung weist die erste Seite des freien Endes des Haltearms keine Auskragung auf. Stattdessen sind befindet sich je eine Auskragung auf den Seitenflächen des Haltearms und der Vorsprung befindet sich auf der zweiten Seite des freien Endes des Haltearms. Die Auskragungen stehen also seitlich in eine Richtung rechtwinklig zur ersten und zweiten Seite ab. Diese Ausführung bietet den Vorteil, dass der Haltearm in radialer Richtung weniger Platz benötigt. Entsprechend kann das Zusatzmodul kompakt konstriert werden.

In beiden Varianten kann durch eine Schwenkbewegung des Haltearms entweder die Auskragung bzw. die Auskragungen in das Gehäuse eingreifen oder der Vorsprung zum eingesetzten Injektor hin verschoben werden, so dass der Vorsprung mit dem Injektor zusammenwirken kann. Der Haltearm ermöglicht somit, dass der Vorsprung einfach und rasch verstellt werden kann. In einer bevorzugten Ausführungsvariante ist der Haltearm zu der mittigen Längsachse der Aufnahmeeinheit bzw. zum eingesetzten Injektor hin elastisch vorgespannt.

Zudem umfasst in einer bevorzugten Ausführung die Aufnahmeeinheit mindestens zwei Haltearme, die einander gegenüberliegend angeordnet sind. Der Injektor kann dadurch besonders sicher im Gehäuse gehalten werden.

Mit Vorteil befindet sich in der Haltekonfiguration das Halteelement in einer Halteposition, in der die Auskragung oder sonst ein Teil des Halteelements durch das Gehäuse verriegelt ist, so dass das Halteelement nicht relativ zur Aufnahmeeinheit bewegt werden kann und in der der Vorsprung in den im Gehäuse eingesetzten Injektor eingreift. Das bedeutet, das Gehäuse verriegelt die Auskragung, so dass das Halteelement nicht mit einer Lösebewegung aus seiner Halteposition vom eingesetzten Injektor wegbewegt werden kann. Der Injektor ist somit in der Haltekonfiguration sicher im Gehäuse des Zusatzmoduls gehalten und gegen unbeabsichtigtes oder spontanes Lösen gesichert.

Die Verriegelung des Halteelements durch das Gehäuse erfolgt vorzugsweise dadurch, dass das Gehäuse oder ein zum Gehäuse gehörendes Element die Auskragung mit einer Fläche berührt, wodurch das Halteelement an einer Bewegung von einer Mitte der Aufnahmeeinheit nach aussen oder vom eingesetzten Injektor weg gehindert wird. Obwohl das Halteelement in der Halteposition unbewegbar verriegelt ist, schliesst dies nicht aus, dass sich das Halteelement durch ein kleines Spiel in einem Bereich, vorzugsweise in einem Bereich kleiner als einen Millimeter, bewegen kann. Dieses allenfalls vorhandene Spiel, stellt sicher, dass das Halteelement nicht in ungewünschter Weise im Gehäuse blockiert wird oder verkantet.

In einer anderen Ausführung kann die Verriegelung des Halteelements beispielsweise dadurch erfolgen, dass die Auskragung in einer Führung im Gehäuse gehalten ist, wodurch sich das Halteelement nicht vom Injektor wegbewegen kann.

Alternativ besteht die Möglichkeit, dass das Halteelement in der Haltekonfiguration nicht verriegelt ist, sondern nur gehalten ist. Entsprechend ist in diesem Fall das Halteelement nicht gegen unbeabsichtigtes oder spontanes Lösen gesichert.

Vorzugsweise befindet sich in der Freigabekonfiguration das Halteelement in einer Freigabeposition, in der die Auskragung in einer Ausnehmung im Gehäuse aufgenommen ist und in der der Vorsprung von einem Innenraum der Aufnahmeeinheit zurückgezogen ist, so dass der Injektor vom Vorsprung freigegeben ist. Vorzugsweise ist in der Freigabeposition die Auskragung so in einer Führung im Gehäuse aufgenommen, dass sie nicht unbeabsichtigt aus dem Gehäuse lösen kann.

In der Freigabeposition ist der Vorsprung vorzugsweise vom Innenraum der Aufnahmeeinheit wegbewegt. Das bedeutet, wenn der Injektor in das Gehäuse eingeführt wird, gelangt er nicht in Berührung mit dem Vorsprung des Halteelements. Das hat den Vorteil, dass der Injektor ohne Widerstand in das Gehäuse einsetzbar ist.

Vorzugsweise ist die Bewegung des Halteelements an die Bewegung der Aufnahmeeinheit gekoppelt. In diesem Fall wird in einer bevorzugten Ausführung durch die Verstellbewegung der Aufnahmeeinheit in eine erste Richtung, um das Zusatzmodul von der Haltekonfiguration in die Freigabekonfiguration zu verstellen, das Halteelement von seiner Halteposition in seine Freigabeposition geführt. Durch diese einzige Verstellbewegung der Aufnahmeeinheit wird bevorzugt der Vorsprung aus einer Aussparung im Injektor zurückgezogen und die Auskragung in eine Ausnehmung im Gehäuse eingeführt.

Zudem wird bevorzugt durch die Verstellbewegung der Aufnahmeeinheit in eine zweite Richtung, um das Zusatzmodul von der Freigabekonfiguration in die Haltekonfiguration zu verstellen, das Halteelement von seiner Freigabeposition in seine Halteposition bewegt. Durch diese einzige Verstellbewegung der Aufnahmeeinheit wird vorzugsweise die Auskragung aus dem Gehäuse freigegeben und der Vorsprung des Halteelements gelangt in Eingriff mit dem im Gehäuse eingesetzten Injektor, wodurch der Injektor unbeweglich an der Aufnahmeeinheit gehalten wird.

In einer bevorzugten Ausführung ist die zweite Richtung entgegengesetzt zur ersten Richtung. Indem mit einer einzigen Verstellbewegung das Zusatzmodul von der Haltekonfiguration in die Freigabekonfiguration oder zurück verstellt werden kann, wird das Verbinden des Injektors mit dem Zusatzmodul und das Trennen des Injektors vom Zusatzmodul für den Benutzer vereinfacht.

Bevorzugt ist das Zusatzmodul nur in die Haltekonfiguration verstellbar, wenn das Halteelement in seine Halteposition bewegbar ist. Das Halteelement ist beispielsweise nicht in seine Halteposition bewegbar, wenn der Vorsprung nicht in den Innenraum der Aufnahmeeinheit bewegbar ist. Das ist zum Beispiel der Fall, wenn ein Injektor ins Gehäuse eingeführt wird, jedoch keine Aussparung für den Vorsprung vorhanden ist und somit der Vorsprung nicht mit dem Injektor zusammenwirken kann. Dieser Fall kann insbesondere dann auftreten, wenn der Injektor oder zumindest die Aussparung für den Vorsprung nicht rotationssymmetrisch ausgestaltet ist und in einer falschen rotativen Ausrichtung in das Gehäuse eingeführt wird.

Das Zusatzmodul, welches nur in die Haltekonfiguration verstellbar ist, wenn das Halteelement in seine Halteposition bewegbar ist, stellt somit sicher, dass der Injektor nicht in einer falschen rotativen Ausrichtung mit dem Zusatzmodul verbunden werden kann, in welcher das Zusatzmodul die Injektionsereignisse im Injektor nicht optimal erkennen kann. Ein solcher Mechanismus kann auch als Verdrehsicherung bezeichnet werden, da der Injektor nur in einer bestimmten rotativen Ausrichtung im Gehäuse gehalten werden kann.

Bevorzugt umfasst die Halteeinrichtung einen Führungsnocken und die Aufnahmeeinheit einen Sperrnocken, wobei in der Haltekonfiguration der Führungsnocken derart mit dem Sperrnocken zusammenwirkt, dass die Aufnahmeeinheit unbeweglich im Gehäuse gehalten ist, entgegen einer möglichen Vorspannung in Richtung einer Längsachse des Gehäuses durch das elastische Element. Das bedeutet, die Aufnahmeeinheit ist durch das Zusammenwirken des Sperrnockens mit dem Führungsnocken unbeweglich am Gehäuse gehalten. Die Aufnahmeeinheit kann demnach nicht ohne vorheriges Lösen des Sperrnockens vom Führungsnocken bewegt werden, um mit der Verstellbewegung das Zusatzmodul von der Haltekonfiguration in die Freigabekonfiguration zu verstellen. Durch eine Sicherung des Zusammenwirkens von Sperr- und Führungsnocken wie zum Beispiel durch eine vorgespannte Feder kann die Aufnahmeeinheit gegen unbeabsichtigtes Verschieben gesichert sein, wodurch der Injektor gegen unbeabsichtigtes Lösen aus dem Gehäuse gesichert ist. Die Nocken beziehungsweise deren Funktionen können auch vertauscht angeordnet sein, das heisst, der bewegliche Führungsnocken kann auch auf der Aufnahmeeinheit angeordnet sein.

Mit Vorteil weisen der Führungsnocken und der Sperrnocken je eine Fläche auf, die miteinander derart in Kontakt stehen, dass der Sperrnocken vom Führungsnocken gehalten ist.

Alternativ dazu besteht auch die Möglichkeit, dass die Halteeinrichtung keinen Führungsnocken und die Aufnahmeeinheit keinen Sperrnocken umfasst. So kann die Halteeinrichtung beispielsweise einen Stift und die Aufnahmeeinheit Kulissenführung umfasst, in der der Stift der Halteeinrichtung geführt wird. Damit können die Bewegungen von einem Element der Halteeinrichtung auf die Aufnahmeeinheit oder umgekehrt mittels einer Kulissensteuerung übertragen werden.

In einer bevorzugten Ausführung ist der Führungsnocken entlang eines Sicherungswegs verschiebbar und ist von einer Haltestellung, in welcher der Führungsnocken mit dem Sperrnocken zusammenwirkt, in eine Freigabestellung, in der der Sperrnocken vom Führungsnocken freigegeben ist, verstellbar. Vorzugsweise befindet sich in der Haltekonfiguration der Führungsnocken der Halteeinrichtung in seiner Haltestellung. In der Freigabekonfiguration hingegen kann sich der Führungsnocken in seiner Freigabestellung, seiner Haltestellung oder in einer weiteren Stellung befinden.

Wird der Führungsnocken aus seiner Haltestellung zu seiner Freigabestellung bewegt, wird er bevorzugt vom Sperrnocken entfernt, wodurch sich die Aufnahmeeinheit frei relativ zum Gehäuse bewegen kann. Dann kann das Zusatzmodul mittels der Verstellbewegung der Aufnahmeeinheit von der Haltekonfiguration in die Freigabekonfiguration und zurück verstellt werden.

Der Sicherungsweg ist vorzugsweise linear. Bevorzugt ist der Sicherungsweg rechtwinklig zur Längsachse des Gehäuses ausgerichtet. In alternativen Ausführungen kann der Sicherungsweg auch gekrümmt oder schraubenlinienförmig sein. In diesem Fall ist der Führungsnocken durch eine Dreh- oder Schraubbewegung zwischen seiner Haltestellung und seiner Freigabestellung bewegbar.

Vorzugsweise ist der Führungsnocken durch ein separates elastisches Sicherungselement in seine Haltestellung vorspannbar und dadurch gegen spontanes Lösen gesichert. Dadurch muss der Führungsnocken gegen eine Vorspannkraft bewegt werden, wenn er entlang des Sicherungswegs von seiner Haltestellung wegbewegt wird, um ein Verstellen der Aufnahmeeinheit und damit ein Verstellen des Zusatzmoduls von der Haltekonfiguration in die Freigabekonfiguration zu ermöglichen. Das elastische Element ist vorzugsweise eine mechanische Feder. Alternativ kann das elastische Element zum Beispiel auch durch einen elastischen Kunststoff gebildet sein.

In einer bevorzugten Ausführung befindet sich der Führungsnocken sowohl in der Haltekonfiguration wie auch in der Freigabekonfiguration in seiner Haltestellung. In diesem Fall muss die Vorspannkraft des elastischen Elements überwunden werden, wenn die Aufnahmeeinheit in eine erste Richtung verstellt wird, um das Zusatzmodul von der Haltekonfiguration in die Freigabekonfiguration zu verstellen wie auch wenn die Aufnahmeeinheit in die zweite Richtung verstellt wird, um das Zusatzmodul von der Freigabekonfiguration in die Haltekonfiguration zu verstellen.

Durch die Vorspannung ist sichergestellt, dass sich der Führungsnocken nicht unbeabsichtigt aus seiner Halteposition lösen kann und damit den Sperrnocken der Aufnahmeeinheit unbeabsichtigt freigibt, wodurch die Aufnahmeeinheit das Zusatzmodul von der Haltekonfiguration in die Freigabekonfiguration verstellen könnte. Somit ermöglicht die Vorspannung ein gesichertes Halten des Injektors im Gehäuse des Zusatzmoduls.

Vorzugsweise befindet sich die Haltestellung an einem ersten Ende des Sicherungswegs, die Freigabestellung in einem mittleren Bereich des Sicherungswegs und eine Lösestellung an einem zweiten Ende des Sicherungswegs. Das bedeutet, der Führungsnocken ist von seiner Haltestellung in seine Freigabestellung und von dieser in seine Lösestellung und zurück bewegbar. In der Lösestellung befindet sich der Führungsnocken vorzugsweise, wenn sich das Zusatzmodul in seiner Freigabekonfiguration befindet.

Mit Vorteil umfasst die Halteeinrichtung einen Löseknopf, mit dem das Zusatzmodul von seiner Haltekonfiguration in seine Freigabekonfiguration verstellbar ist. Dadurch kann auf einfache Art und Weise der Injektor aus dem Gehäuse des Zusatzmoduls freigegeben werden. Vorzugsweise wird beim Betätigen des Löseknopfs der Führungsnocken der Halteeinrichtung aus seiner Haltestellung entlang dem Sicherungsweg zu seiner Freigabeposition bewegt, wodurch der Sperrnocken der Aufnahmeeinheit freigegeben wird und dadurch die Aufnahmeeinheit relativ zum Gehäuse bewegbar ist.

Alternativ umfasst die Halteeinrichtung keinen Löseknopf und die Aufnahmeeinheit muss durch den Benutzer manuell bewegt werden, um das Zusatzmodul von der Haltekonfiguration in die Freigabekonfiguration verstellen zu können.

In einer bevorzugten Ausführung ist die Halteeinrichtung mit dem Löseknopf sowie das Halteelement in einem distalen Bereich des Gehäuses des Zusatzmoduls angeordnet. Dabei entspricht der distale Bereich einem Bereich des Gehäuses, welcher dem einstechseitigen Bereich des eingesetzten Injektors zugewandt ist. Dadurch befinden sich die Halteeinrichtung mit dem Löseknopf und das Halteelement im Bereich einer Öffnung des Gehäuses und der proximale Bereich des Gehäuses bleibt frei, so dass der Benutzer das Zusatzmodul im proximalen Bereich gut ergreifen kann.

Diese Anordnung hat zudem den Vorteil, dass der Löseknopf vom Benutzer nicht irrtümlicherweise als Knopf zum Aktivieren des Ausschüttens aufgefasst wird, da sich üblicherweise solche Knöpfe zum Ausschütten in einem proximalen Endbereich befinden.

Zudem betrifft die Erfindung ein Verfahren zum Verbinden eines Injektors mit einem Zusatzmodul, insbesondere mit einem Zusatzmodul wie vorhergehend beschrieben. Das Verfahren umfasst die Schritte
a. Einführen des Injektors in das Zusatzmodul mit einer Einführbewegung;
b. Verstellen, durch das Einführen, einer Aufnahmeeinheit im Gehäuse des Zusatzmoduls relativ zum Gehäuse,
c. Bewegen, durch das Verstellen, des Halteelements zum Injektor hin, so dass das Halteelement mit dem Injektor zusammenwirkt und dadurch der Injektor in der Aufnahmeeinheit gehalten wird;
d. Sichern der Aufnahmeeinheit am Zusatzmodul durch die Halteeinrichtung.

Vorzugsweise wird das Halteelement durch das Gehäuse verriegelt, durch das Verstellen der Aufnahmeeinheit.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden.
- Fig. 1: zeigt eine perspektivische Ansicht eines Zusatzmoduls, welches mit einem Injektor verbunden ist;
- Fig. 2: zeigt die Einzelteile des Zusatzmoduls;
- Fig. 3: zeigt eine Schnittansicht des Zusatzmoduls in der Freigabekonfiguration ohne eingesetzten Injektor;
- Fig. 4: zeigt eine Schnittansicht eines mittleren Bereichs des Zusatzmoduls, in welchem Bereich die Halteeinrichtung in der Freigabekonfiguration ersichtlich ist;
- Fig. 5: zeigt eine Schnittansicht des Zusatzmoduls in der Haltekonfiguration mit eingesetztem Injektor;
- Fig. 6: zeigt eine Schnittansicht eines mittleren Bereichs des Zusatzmoduls, in welchem Bereich die Halteeinrichtung in der Haltekonfiguration ersichtlich ist;
- Fig. 7: zeigt eine Schnittansicht des Zusatzmoduls mit einem nicht korrekt eingesetzten Injektor;
- Fig. 8: zeigt eine perspektivische Ansicht einer weiteren Ausführung des Zusatzmoduls in der Haltekonfiguration;
- Fig. 9: zeigt eine Detailansicht der weiteren Ausführung aus Figur 8, wobei die obere Hälfte des Gehäuseeinsatzes und des Löseknopfs nicht dargestellt ist;
- Fig. 10: zeigt eine perspektivische Ansicht einer Ausführung des Zusatzmoduls in der Haltekonfiguration mit einer konkaven Gehäuseform;
- Fig. 11: zeigt eine Ansicht von unten der Ausführung aus Figur 10;
- Fig. 12: zeigt eine Ansicht der Ausführung aus Figur 10 in der Freigabekonfiguration.

### FIGURENBESCHREIBUNG

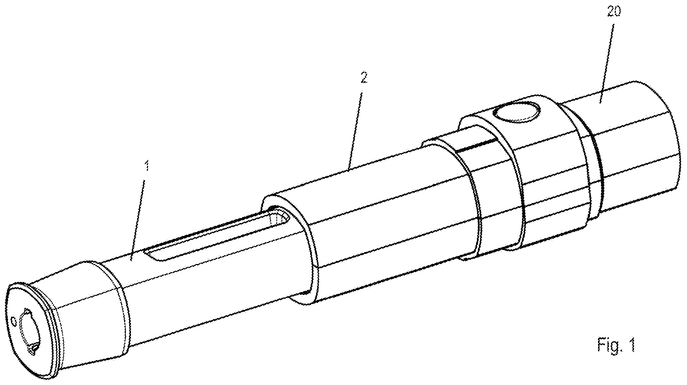

Die Figur 1 zeigt in einer perspektivischen Ansicht ein erfindungsgemässes Zusatzmodul 2, welches mit einem Injektor 1, im gezeigten Ausführungsbeispiel ist es ein Autoinjektor, verbunden ist. In der vorliegenden Beschreibung wird beim Zusatzmodul 2 das Ende des Zusatzmoduls 2, welches die Öffnung zu einem Innenraum umfasst, als distales Ende (in Figur 1 linke Seite) und das hintere, geschlossene Ende des Zusatzmoduls 2 als proximales Ende (in Figur 1 rechte Seite) bezeichnet. Beim Injektor 1 wird das einstechseitige Ende als distales Ende und das hintere, dem einstechseitigen Ende gegenüberliegende Ende als proximales Ende bezeichnet.

Das Zusatzmodul 2 ist am proximalen Ende des Injektors 1 angebracht, wobei der proximale Bereich des Injektors 1 vollständig vom Zusatzmodul 2 umschlossen wird. Der distale Bereich des Injektors 1 hingegen ist nicht vom Zusatzmodul 2 umgeben.

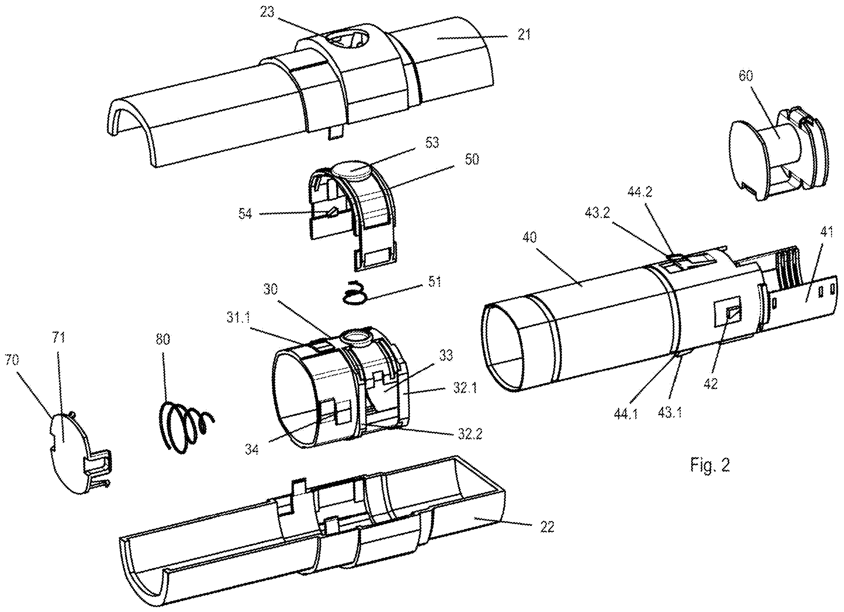

Die Figur 2 stellt die Einzelteile des erfindungsgemässen Zusatzmoduls 2 dar. Diese werden nachfolgend erläutert. Das Zusatzmodul 2 umfasst ein zweistückig ausgebildetes Gehäuse 20 mit einer oberen Gehäusehälfte 21 und einer unteren Gehäusehälfte 22, ein zum Gehäuse 20 gehörender Gehäuseeinsatz 30, eine Aufnahmeeinheit 40 mit einem Anschlagelement 70 und einer Druckfeder 80, ein Löseknopf 50 mit einer Löseknopffeder 51 und ein Elektronikmodul 60.

Die obere und die untere Gehäusehälfte 21, 22 weisen je eine halbrunde Form auf und sind miteinander mittels Ultraschallschweissen verbunden. Alternativ ist auch ein Verbinden mittels Laserschweissen oder Kleben möglich. Durch die halbrunde Form der Gehäusehälften 21, 22 entsteht zwischen ihnen ein Innenraum. An einem proximalen Ende der Gehäusehälften 21, 22 weisen diese je eine Abschlusswandung auf, so dass der Innenraum am proximalen Ende durch diese Abschlusswandung begrenzt wird. Am distalen Ende sind die Gehäusehälften 21, 22 offen. Somit hat das Gehäuse 20 am distalen Ende eine Öffnung zum Innenraum. Im Innenraum befinden sich der Gehäuseeinsatz 30, der Löseknopf 50 und die Aufnahmeeinheit 40. In proximaler Richtung hinter dem Gehäuseeinsatz 30 ist das Elektronikmodul 60 untergebracht.

Die halbrunde obere Gehäusehälfte 21 weist in einem mittleren Bereich der halbrunden Form einen Öffnung 23 auf, durch die im montierten Zustand eine Betätigungsfläche 53 des Löseknopfs 50 hindurch ragt. In Bezug auf den Querschnitt an äusseren Enden weist die obere Gehäusehälfte 21 je ein Schnapparm auf, welche beim Verbinden mit der unteren Gehäusehälfte 22 in entsprechend Ausnehmungen in der unteren Gehäusehälfte 22 einschnappen. Ebenso weist die halbrunde untere Gehäusehälfte 22 Schnapparme auf, welche in Ausnehmungen in der oberen Gehäusehälfte 21 einschnappen beim Verbinden der Gehäusehälften 21, 22. Durch diese Schnappverbindung können die Gehäusehälften 21, 22 vor dem Ultraschallschweissen vormontiert werden. Die beiden Gehäusehälften 21, 22 bilden das Gehäuse 20 des Zusatzmoduls 2. Das Gehäuse 20 weist im Querschnitt näherungsweise eine quadratische Form auf, wobei jede Seite des Quadrats leicht nach aussen gewölbt ist.

In der nachfolgenden Beschreibung wird der Einfachheit halber die Richtung von der mittigen Längsachse des Gehäuses 20 nach aussen zu den Seitenwänden des Gehäuses 20 als radiale Richtung bezeichnet, obwohl das Gehäuse 20 in der gezeigten Ausführung keinen kreisrunden Querschnitt aufweist.

Der Gehäuseeinsatz 30 hat eine hülsenförmige Gestalt. In zwei Aussenseiten weist der Gehäuseeinsatz 30 je eine Öffnung 31.1 auf, welche je in einem spitzen Winkel zur Längsachse des Gehäuseeinsatzes 30 von einer Aussenseite in proximaler Richtung durch die Wandung des Gehäuseeinsatzes 30 nach innen verläuft. Der Gehäuseeinsatz 30 weist zudem auf zwei Aussenseiten je ein Durchbruch 33 in der Wandung auf, durch welche Führungsnocken 54 des Löseknopfs 50 mit Sperrnocken 42 der Aufnahmeeinheit 40 zusammenwirken können. Weiter lagert der Gehäuseeinsatz 30 verschiebbar den Löseknopf 50. Hierzu umfasst der Gehäuseeinsatz 30 auf zwei Aussenseiten je zwei Auflageflächen 32.1, 32.2. Der Gehäuseeinsatz 30 ist in einer radialen Vertiefung im Gehäuse 20 aufgenommen, wodurch der Gehäuseeinsatz 30 relativ zum Gehäuse 20 unbeweglich fixiert ist. In Umfangsrichtung ist der Gehäuseeinsatz 30 über je zwei auf zwei Aussenseiten des Gehäuseeinsatzes 30 angebrachte Rippen 34, welche in entsprechende Nuten in der Innenwand des Gehäuses 20 eingreifen, relativ zum Gehäuse 20 unbeweglich gehalten.

Der Löseknopf 50 hat einen U-förmigen Querschnitt und ist auf dem Gehäuseeinsatz 30 gelagert, so dass die Schenkel der U-Form den Gehäuseeinsatze 30 beidseitig umgeben und auf den Auflageflächen 32.1, 32.2 aufliegen. Zwischen den Schenkeln weist der Löseknopf 50 auf einer Aussenseite die Betätigungsfläche 53 auf, die der Benutzer mit der Hand betätigen kann, um den Löseknopf 50 zu bewegen. Ferner weisen die Schenkel je auf ihrer Innenseite einen dreieckförmigen Führungsnocken 54 auf, welche je mit einem dreieckförmigen Sperrnocken 42 der Aufnahmeeinheit 40 zusammenwirken können und dadurch eine Halteeinrichtung für die Aufnahmeeinheit 40 bilden. Die Funktion der Führungsnocken 54 wird weiter unten im Detail erklärt.

Der Löseknopf 50 befindet sich in radialer Richtung zwischen Gehäuseeinsatz 30 und Gehäuse 20. Dabei kann sich der Löseknopf radial, in eine Richtung rechtwinklig zur Längsachse des Gehäuses 20, relativ zum Gehäuseeinsatz 30 und zum Gehäuse 20 entlang einem linearen Sicherungsweg bewegen. Zwischen dem Gehäuseeinsatz 30 und dem Löseknopf 50 befindet sich die Löseknopffeder 51. Diese spannt den Löseknopf 50 radial nach aussen vor.

Im Innenraum des Gehäuses 20 befindet sich die Aufnahmeeinheit 40. Diese ist in axialer Richtung relativ zum Gehäuse 20 und damit relativ zum Gehäuseeinsatz 30 bewegbar gelagert. Die Aufnahmeeinheit 40 ist ebenfalls hülsenförmig ausgeführt und hat im Querschnitt im Wesentlichen eine quadratische Form, wobei die Seitenwände des Quadrats jeweils leicht nach aussen gewölbt sind.

In einer Ausführung weist die Aufnahmeeinheit 40 in ihrem Innenraum in axialer Richtung verlaufende Stege oder Rippen auf (nicht dargestellt). Dadurch liegt ein in den Innenraum der Aufnahmeeinheit 40 eingeschobener Injektor auf den Stegen oder Rippen auf. Druckempfindliche Elemente des Injektos wie beispielsweise elektronische Elemente oder NFC-Tags auf der Aussenseite des Injektor können dadurch in den Zwischenräumen zwischen den Stegen oder Rippen aufgenommen werden. Sie sind somit geschützt und werden nicht an die Innenwand des Innenraums der Aufnahmeeinheit gedrückt.

In einem proximalen Bereich weist die Aufnahmeeinheit 40 einen Abschnitt 41 mit einer halbrunden Form im Querschnitt auf. In diesem Abschnitt 41 ist das Elektronikmodul 60 aufgenommen. Somit ist das Elektronikmodul 60 zusammen mit der Aufnahmeeinheit 40 relativ zum Gehäuse bewegbar.

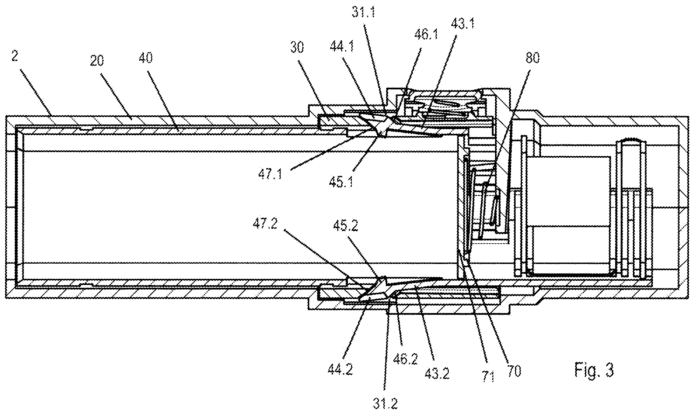

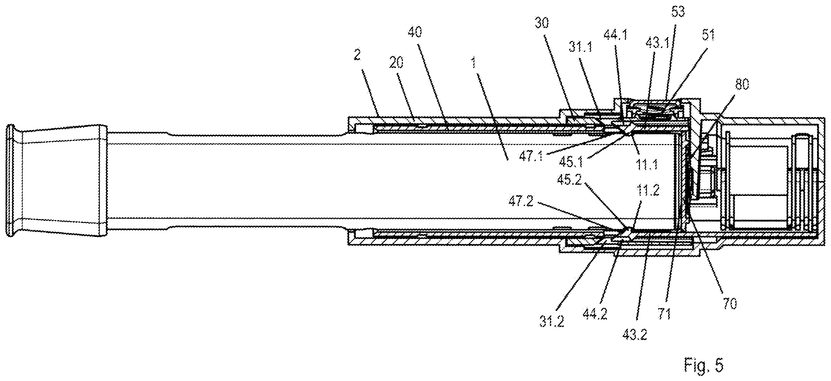

Auf zwei einander gegenüberliegenden Seiten der quadratischen Form der Aufnahmeeinheit 40 ist je ein Halteelement in der Form eines Haltearms 43.1, 43.2 angeordnet, wobei die Haltearme 43.1, 43.2 sich in Bezug auf die axiale Länge der Aufnahmeeinheit 40 in einem mittleren Bereich der Aufnahmeeinheit 40 befinden. Die Haltearme 43.1. 43.2 sind an einem ersten Ende schwenkbar mit der Aufnahmeeinheit 40 verbunden. Im Bereich des freien Endes auf einer dem Innenraum abgewandten Aussenseite der Haltearme 43.1, 43.2 weisen diese je eine Auskragung 44.1, 44.2 auf, welche je in den Öffnungen 31.1 aufgenommen werden können. An den Auskragungen 44.1, 44.2 sind je eine distale und eine proximale Gleitfläche ausgebildet. Diese werden weiter unten im Zusammenhang mit den Figuren 3 und 5 näher beschreiben. Ebenfalls im Bereich des freien Endes der Haltearme 43.1, 43.2 jedoch auf einer Innenseite, welche dem Innenraum der Aufnahmeeinheit 40 zugewandt ist, weisen die Haltearme 43.1, 43.2 je einen Vorsprung auf. Auf den anderen zwei Seiten der quadratischen Form ist je ein dreieckförmiger Sperrnocken 42 angeordnet, welche je mit einem Führungsnocken 54 des Löseknopf 50 zusammenwirken können.

Weiter umfasst die Aufnahmeeinheit 40 in ihrem Innenraum das Anschlagelement 70 mit einer Anschlagfläche 71, die rechtwinklig zur Längsachse der Aufnahmeeinheit 40 ausgerichtet ist und sich über den ganzen Querschnitt des Innenraums der Aufnahmeeinheit 40 erstreckt. Das Anschlagelement 70 ist dabei in einem proximalen Bereich der Aufnahmeeinheit 40 positioniert. Zudem ist das Anschlagelement 70 unbewegbar mittels einer Schnappverbindung mit der Aufnahmeeinheit 40 verbunden.

Zwischen dem Anschlagelement 70 und einer rechtwinklig zur Längsachse des Gehäuses ausgerichteten Wandung des Gehäuses, welche in den Innenraum des Gehäuses ragt, befindet sich die Druckfeder 80. Über das Anschlagelement 70 spannt diese Druckfeder 80 die Aufnahmeeinheit 40 in distaler Richtung vor.

### Freigabekonfiguration des Zusatzmoduls

Nachfolgend wird auf die Funktion des Zusatzmoduls im Detail eingegangen. In der Figur 3 ist eine Schnittansicht des Zusatzmoduls 2 in einer Freigabekonfiguration ersichtlich, wobei der Schnitt durch die Längsachse des Zusatzmoduls 2 verläuft.

Wie erwähnt, ist die Aufnahmeeinheit 40 im Gehäuse 20 in axialer Richtung verschiebbar gelagert. Wie in Figur 3 ersichtlich, befindet sich die Aufnahmeeinheit 40 in der Freigabekonfiguration an einem distalen Ende ihres Verstellwegs. Die Aufnahmeeinheit 40 wird in diese Position durch die Druckfeder 80 vorgespannt. Wenn also die Aufnahmeeinheit 40 in proximaler Richtung relativ zum Gehäuse 20 verschoben werden soll, muss sie gegen die Vorspannkraft der Druckfeder 80 bewegt werden. Die Aufnahmeeinheit 40 kann soweit in proximaler Richtung relativ zum Gehäuse 20 verschoben werden bis ein proximales Ende der Aufnahmeeinheit 40 an der proximalen Abschlusswandung des Gehäuses 20 anschlägt.

In Figur 3 ist zudem ersichtlich, dass die beiden Haltearme 43.1, 43.2 vom Körper der Aufnahmeeinheit 40 nach aussen abstehen, indem jeweils die Auskragung 44.1, 44.2 der Haltearme 43.1, 43.2 in den Öffnungen 31.1, 31.2 im Gehäuseeinsatz 30 aufgenommen sind und in diesen zwangsgeführt sind. Da die Haltearme 43.1, 43.2 dadurch nach aussen weisen, ragen die Vorsprünge 45.1, 45.2 auf der jeweiligen Unterseite der Haltearme 43.1, 43.2 nicht in den Innenraum der Aufnahmeeinheit 40, sondern sind im Wesentlichen bündig mit der Innenfläche der Aufnahmeeinheit 40. In dieser Stellung befinden sich die Haltearme 43.1, 43.2 in einer Freigabeposition.

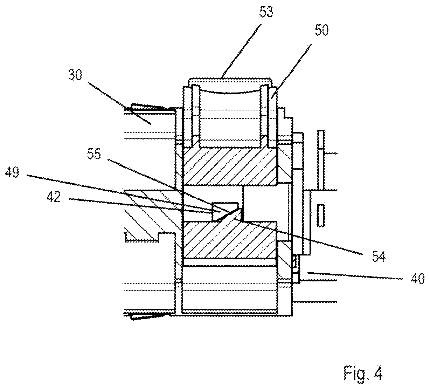

Wie oben erwähnt, weist die Aufnahmeeinheit 40 auf zwei Aussenseiten je einen Sperrnocken 42 auf. Der Löseknopf 50 umfasst auf seiner Innenseite zwei einander gegenüberliegende Führungsnocken 54, ersichtlich in Figur 4. Die Figur 4 zeigt einen mittleren Bereich des Zusatzmoduls 2 in der Freigabekonfiguration, wobei die äussere Wandung des Gehäuses 20 und des Gehäuseeinsatzes 30 und ein Schenkel der U-Form des Löseknopfs 50 in der Darstellung weggeschnitten sind, damit einer der Führungsnocken 54 und einer der Sperrnocken 42 ersichtlich sind.

In der Freigabekonfiguration befinden sich die Sperrnocken 42 auf der distalen Seite der Führungsnocken 54. Die Sperrnocken 42 umfassen je eine Kontaktfläche 49, welche in einem spitzen Winkel zur Längsachse des Gehäuses 20 steht. Die Führungsnocken 54 umfassen je eine Führungsfläche 55, die ebenfalls in diesem Winkel zur Längsachse ausgerichtet sind. In der Freigabekonfiguration, in der sich die Aufnahmeeinheit 40 an einem distalen Ende ihres Verstellwegs befindet, befinden sich die Führungsnocken 54 in einer Lösestellung, in der die Kontaktflächen 49 der Sperrnocken 42 und die Führungsflächen 55 der Führungsnocken 54 aufeinander liegen. Die Führungsfläche 55 ist radial nach aussen vorgespannt, da der Löseknopf 50 durch die Löseknopffeder 51 vorgespannt ist.

### Verstellung von der Freigabekonfiguration in die Haltekonfiguration

Zum Verbinden des Injektors 1 mit dem Zusatzmodul 2, muss das Zusatzmodul 2 von der Freigabekonfiguration in eine Haltekonfiguration verstellt werden. Hierzu muss die Aufnahmeeinheit 40 relativ zum Gehäuse 20 von einer distalen Endposition in eine proximale Endposition verschoben werden.

Diese Verschiebung erfolgt mittels des Injektors 1, indem dieser mit seinem proximalen Ende in die Öffnung am distalen Ende des Gehäuses 20 eingeschoben und in Richtung proximales Ende des Gehäuses 20 bewegt wird. Hierbei schlägt das eingeführte Ende des Injektors 1 an der Anschlagfläche 71 des Anschlagelements 70 an. Wird der Injektor 1 weiter in proximaler Richtung in das Gehäuse 20 eingeschoben, wird das Anschlagelement 70 und die mit dem Anschlagelement 70 verbundene Aufnahmeeinheit 40 gegen die Vorspannkraft der Druckfeder 80 in proximaler Richtung verschoben, wodurch sich die Druckfeder 80 komprimiert. Bei dieser Verschiebung werden auch die Haltearme 43.1, 43.2 in proximaler Richtung mitbewegt, wodurch die Auskragungen 44.1, 44.2 der Haltearme 43.1, 43.2 jeweils aus den schrägen Öffnungen 31.1, 31.2 im Gehäuseeinsatz 30 heraus gleiten und sich dadurch zum Innenraum des Gehäuses 20 hin bzw. zum eingesetzten Injektor 1 hin bewegen. Dadurch bewegen sich jeweils auch die Vorsprünge 45.1, 45.2 an den Unterseiten der Haltearme 43.1, 43.2 zum Injektor 1 hin und greifen mit zunehmender Verschiebung der Aufnahmeeinheit 40 in proximaler Richtung in Aussparungen 11.1, 11.2 oder Nuten im Injektor 1 ein.

Bei dieser Bewegung der Haltearme 43.1, 43.2 gleiten jeweils die proximalen Gleitflächen 46.1, 46.2 der Auskragungen 44.1, 44.2 entlang einer Innenfläche der Öffnungen 31.1, 31.2 bis sie das Ende der Öffnung 31.1, 31.2 erreicht haben. Die Auskragungen 44.1, 44.2 gleiten in diesem Moment aus den Öffnung 31.1, 31.2 heraus und werden mit weiterer Verschiebung der Haltearme 43.1, 43.2 in proximaler Richtung durch eine Innenwandung des Gehäuseeinsatzes 30 nach innen zum Innenraum hin gezwungen. Dadurch werden die Vorsprünge 45.1, 45.2 weiter zum eingesetzten Injektor 1 hin gedrückt und greifen in die Aussparungen 11.1, 11.2 im Injektor 1 ein. Beim weiteren proximalen Verschieben der Aufnahmeeinheit 40 werden die Auskragungen 44.1, 44.2 weiter in den Gehäuseeinsatz 30 hineingezogen. Das bedeutet, die Auskragungen 44.1, 44.2 werden durch die Innenwandung des Gehäuseeinsatzes 30 geführt, so dass die Haltearme 43.1, 43.2 nicht mehr in radialer Richtung nach aussen verschwenkt werden können. Die Haltearme 43.1, 43.2 sind somit durch den Gehäuseeinsatz 30 verriegelt und die Vorsprünge 45.1, 45.2 können sich nicht unbeabsichtigt aus den Aussparungen 11.1, 11.2 im Injektor 1 lösen. Die Haltearme 43.1, 43.2 befinden sich nun in einer Halteposition.

Der Injektor kann auf seiner Aussenseite ein Etikett oder Aufkleber aufweisen, von dem die Aussparungen 11.1, 11.2 überdeckt werden. In diesem Fall kann das Etikett oder der Aufkleber über der Aussparung 11.1, 11.2 H-förmige Einschnitte aufweisen, um das Eingreifen der Vorsprünge 45.1, 45.2 in die Aussparungen 11.1, 11.2 zu erleichtern.

Bei der Verschiebung der Aufnahmeeinheit von der distalen Endposition in die proximale Endposition wirken auch die Sperrnocken 42 der Aufnahmeeinheit 40 mit den Führungsnocken 54 des Löseknopfs zusammen. Sobald nämlich die Aufnahmeeinheit 40 von ihrer distalen Endposition in proximaler Richtung verschoben wird, drücken jeweils die schrägen Kontaktflächen 49 der Sperrnocken 42 der Aufnahmeeinheit 40 gegen die schrägen Führungsflächen 55 der Führungsnocken 54 des Löseknopfs 50. Da sich der Löseknopf 50 nicht in axialer Richtung bewegen kann, jedoch in radialer Richtung bewegbar gelagert ist, verschieben sich die Führungsnocken 54 durch den Druck von den Sperrnocken 42 entgegen der Vorspannkraft der Löseknopffeder 51 in radialer Richtung. Der Löseknopf 50 wird dadurch zum Gehäuse 20 hin bewegt. Anders formuliert, erzeugt der Druck von den Sperrnocken 42 auf die angewinkelten Führungsflächen 55 eine Bewegung der Führungsnocken 54 und damit des Löseknopfs 50 in eine radiale Richtung rechtwinklig zur Verschieberichtung der Aufnahmeeinheit 40. Diese Bewegung des Löseknopfs 50 muss gegen die Federkraft der Löseknopffeder 51 erfolgen und die Löseknopffeder 51 komprimiert sich entsprechend. Durch weiteres Einschieben des Injektors 1 und dadurch weiteres Verschieben der Aufnahmeeinheit 40 in proximaler Richtung gleitet die schrägen Kontaktflächen 49 der Sperrnocken 42 entlang den Führungsflächen 55 und drücken somit den Löseknopf 50 weiter radial zum Gehäuse 20 hin bis die Sperrnocken 42 über die Führungsflächen 55 hinausgleiten und dadurch die Führungsnocken 54 freigeben. In diesem Moment springt der Löseknopf 50 durch die Federkraft der Löseknopffeder 51 wieder radial nach aussen vom Gehäuse 20 weg. Die Sperrnocken 42 befinden sich dann auf der proximalen Seite der Führungsnocken 54. Die Führungsnocken 54 befinden sich in dieser Position in ihrer Haltestellung, in der sie mit einer rechtwinklig zur Längsachse ausgerichteten Verriegelungsfläche 56 die Sperrnocken 42 an einer Bewegung in distaler Richtung hindert.. In dieser Position befindet sich die Aufnahmeeinheit 40 an einem proximalen Ende ihres Verstellwegs und das Zusatzmodul 2 befindet sich in der Haltekonfiguration.

Dieses Verstellen der Führungsnocken 54 von ihrer Lösestellung in ihre Haltestellung erfolgt gleichzeitig mit dem Verstellen der Haltearme 43.1, 43.2 von ihrer Freigabeposition in ihre Halteposition während der Verstellbewegung der Aufnahmeeinheit 40 vom distalen Ende des Verstellwegs zum proximalen Ende des Verstellwegs der Aufnahmeeinheit 40.

### Haltekonfiguration des Zusatzmoduls

Die Figur 5 zeigt das Zusatzmodul 2 in einer Schnittansicht in der Haltekonfiguration mit einem im Gehäuse 20 gehaltenen Injektor 1. Dieser berührt mit seiner Stirnfläche die Anschlagfläche 71 des Anschlagelements 70, wobei sich das Anschlagelement 70 zusammen mit der Aufnahmeeinheit 40 am proximalen Ende des Verstellwegs der Anschlageinheit 40 befindet. Die Druckfeder 80 ist in dieser Position der Aufnahmeeinheit 40 vollständig komprimiert. Durch das oben beschriebene Zusammenwirken von Sperrnocken 42 und Führungsnocken 54 wird die Aufnahmeeinheit 40 in dieser durch die Druckfeder 80 vorgespannten Position gehalten. Die Haltearme 43.1, 43.2 befinden sich in ihrer Halteposition, in der jeweils die Vorsprünge 45.1, 45.2 in die Aussparungen 11.1, 11.2 im Injektor 1 eingreifen und in der die Innenwandung die Auskragungen 44.1, 44.2 der Haltearme 43.1, 43.2 verriegelt. Somit ist der Injektor 1 in der Haltekonfiguration unbewegbar im Gehäuse 20 gehalten.

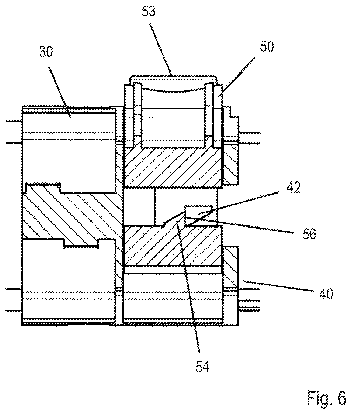

Die Figur 6 zeigt einen mittleren Abschnitt des Zusatzmoduls in der Haltekonfiguration. Dabei ist wie in Figur 4 eine äussere Wandung des Gehäuses 20 und des Gehäuseeinsatzes 30, sowie ein Teil des Schenkels des Löseknopfs 50 weggeschnitten, damit einer der Führungsnocken 54 des Löseknopfs 50 und einer der Sperrnocken 42 der Aufnahmeeinheit 40 ersichtlich sind. In der Figur 6 ist der Führungsnocken 54 in seiner Haltestellung gezeigt. In dieser hindert der Führungsnocken 54 mit seiner Verriegelungsfläche 56 und durch die mit der Löseknopffeder 51 erzeugten Vorspannkraft des Löseknopfs 50 den Sperrnocken 42 und damit die Aufnahmeeinheit 40 an einer Bewegung in eine distale Richtung. Somit ist ein unerwünschtes Verschieben der Aufnahmeeinheit 40 nicht möglich und der Injektor 1 kann sich nicht unbeabsichtigt aus dem Gehäuse 20 des Zusatzmoduls 2 lösen. Da sich in der Haltekonfiguration die Aufnahmeeinheit 40 ganz am proximalen Ende ihres Verstellwegs befindet, kann sie auch nicht weiter in proximaler Richtung verschoben werden.

### Verstellen von der Haltekonfiguration in die Freigabekonfiguration

Um den Injektor 1 vom Zusatzmodul 2 zu trennen, muss das Zusatzmodul 2 von der Haltekonfiguration in die Freigabekonfiguration verstellt werden. Dies wird erreicht, indem die Betätigungsfläche 53 des Löseknopfs 50 gedrückt wird, so dass sich der Löseknopf entlang seines Sicherungswegs in radialer Richtung zum Gehäuse 20 hin verschiebt. Dadurch wird die Löseknopffeder 51 komprimiert und die Führungsnocken 54 verschieben sich von ihrer Haltestellung in eine Freigabestellung, wodurch die Sperrnocken 42 der Aufnahmeeinheit 40 freigegeben werden und in distaler Richtung bewegt werden können.

Dadurch kann sich die in der Haltekonfiguration komprimierte Druckfeder 80 entspannen und drückt dadurch auf das Anschlagelement 70, wodurch das Anschlagelement 70 und die Aufnahmeeinheit 40 in distaler Richtung verschoben werden.

Die Betätigungsfläche 53 muss dabei nur kurzzeitig gedrückt werden, da sich durch die Vorspannkraft der Druckfeder 80 die Aufnahmeeinheit 40 sofort in distaler Richtung bewegt, so dass sich die Sperrnocken 42 zur distalen Seite der Führungsnocken 54 hin verschieben. Wird die Betätigungsfläche 53 losgelassen, verschiebt sich durch die Federkraft der Löseknopffeder 51 der Löseknopf 50 wieder radial nach aussen, wodurch die Führungsnocken 54 nach oben in ihre Lösestellung verschoben werden.

Diese Verschiebung der Aufnahmeeinheit 40 in distaler Richtung bewirkt, dass die Haltearme 43.1, 43.2 ebenfalls in distaler Richtung verschoben werden. Die Auskragungen 44.1, 44.2 führen dabei entlang der Innenwandung des Gehäuseeinsatzes 30 bis sich die Auskragungen 44.1, 44.2 je über dem Eingang der Öffnungen 31.1, 31.2 befinden. Beim weiteren Verschieben der Aufnahmeeinheit 40 in distaler Richtung, werden die Auskragungen 44.1, 44.2 bedingt durch ihre Form in die Öffnungen 31.1, 31.2 hineingeführt und in den Öffnungen 31.1, 31.2 zwangsgeführt. Die distalen Gleitflächen 47.1, 47.2 der Auskragungen 44.1, 44.2 gleiten dann je entlang der Innenfläche der Öffnung 31.1, 31.2, wodurch die Haltearme 43.1, 43.2 schräg nach aussen vom Innenraum der Aufnahmeeinheit 40 wegbewegt werden. Dadurch werden auch die Vorsprünge 45.1, 45.2 vom Innenraum bzw. vom Injektor 1 wegbewegt, wodurch der Injektor 1 von den Haltearmen 43.1, 43.2 freigegeben wird.

Durch die Kraft der Druckfeder 80 wird die Aufnahmeeinheit 40 bis zum distalen Ende ihres Verstellwegs verschoben, bei dem sie an einem distalen Anschlag am Gehäuse 20 anschlägt. Während dieser Bewegung der Aufnahmeeinheit 40 werden die freien Ende der Haltearme 43.1, 43.2 weiter in die jeweiligen Öffnungen 31.1, 31.2 im Gehäuseeinsatz 30 hineingeschoben bis sich die Haltearme 43.1. 43.1 in ihrer Freigabeposition befinden. Während der gesamten Verschiebung der Aufnahmeeinheit 40 verschiebt die Anschlagfläche 71 den Injektor 1 zusammen mit der Aufnahmeeinheit 40 in distaler Richtung, so dass dieser einfacher aus dem Gehäuse entnommen werden kann. Wenn die Aufnahmeeinheit 40 am distalen Ende ihres Verstellwegs angelangt ist, befindet sich das Zusatzmodul in der Freigabekonfiguration und der Injektor 1 kann aus dem Gehäuse 20 entnommen werden.

### Verdrehsicherung

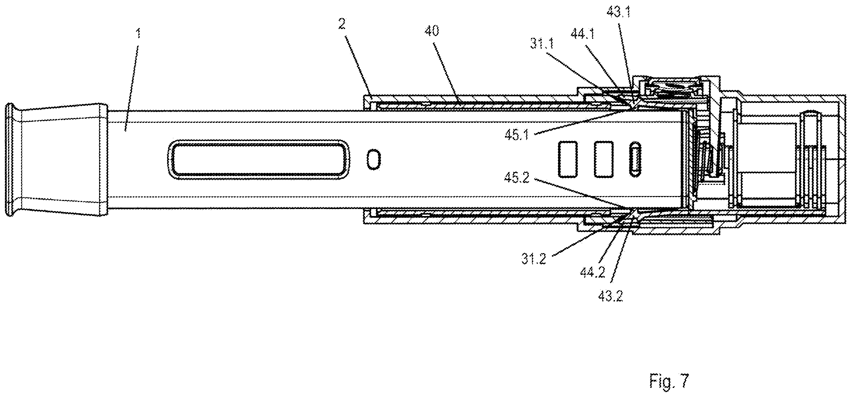

In der Figur 7 ist das Zusatzmodul 2 in einer Schnittansicht mit eingesetzten Injektor 1 ersichtlich. Allerdings ist im Unterschied zur in Figur 5 gezeigten Darstellung der Injektor 1 nicht korrekt eingesetzt. Der gezeigte Injektor 1 umfasst nämlich nur auf zwei Seiten Aussparungen 11.1, 11.2, in die die Vorsprünge 45.1, 45.2 der Haltearme 43.1, 43.2 eingreifen können. In der in Figur 7 gezeigten Position des Injektors 1 sind diese Aussparungen 11.1, 11.2 jedoch nicht zu den Vorsprüngen 45.1, 45.2 ausgerichtet. Wie in der Figur 7 ersichtlich, kann der Injektor 1 zwar in das Gehäuse eingeführt werden, jedoch lässt sich die Aufnahmeeinheit 40 nicht vollständig in die proximale Endposition verschieben.

Da die Vorsprünge 45.1 , 45.2 nicht in die Aussparungen 11.1, 11.2 im Injektor 1 eingreifen können, sondern auf einer Aussenfläche des Injektors 1 anstehen, können die Haltearme 43.1, 43.2 nicht in ihre Halteposition bewegt werden und bleiben nach aussen verschwenkt. Dadurch können die Auskragungen 44.1, 44.2 der Haltearme 43.1, 43.2 nicht vollständig aus den Öffnungen 31.1, 31.2 herausbewegt werden. Die Auskragung 44.1, 44.2 blockieren dadurch die Aufnahmeeinheit 40, so dass diese nicht weiter in proximaler Richtung verschiebbar ist. Somit kann der Sperrnocken nicht den Führungsnocken aus dessen Lösestellung bewegen. Das bedeutet, das Zusatzmodul 2 kann in der in Figur 7 gezeigten Lage des Injektors 1 nicht in seine Haltekonfiguration überführt werden und der Injektor 1 kann somit nicht im Gehäuse 20 gehalten werden.

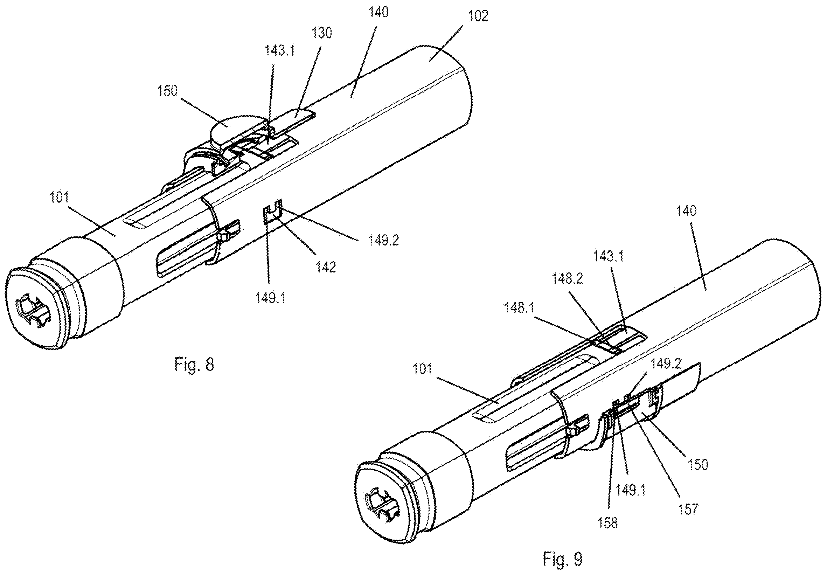

In den Figuren 8 und 9 ist eine weitere Ausführung des erfindungsgemässen Zusatzmoduls 102, welches mit einem Injektor 101 verbunden ist dargestellt.

Die Figuren 8 und 9 zeigen das Zusatzmodul 102 je in der Haltekonfiguration. In der Darstellung in Figur 8 ist seitlich eine Hälfte des Löseknopfs und des Gehäuseeinsatzes darstellerisch weggeschnitten, damit der Haltearm 143.1 besser ersichtlich ist. Zudem ist der besseren Übersichtlichkeit halber die Löseknopffeder nicht dargestellt. In Figur 9 ist die obere Hälfte des Löseknopfs und des Gehäuseeinsatzes darstellerisch weggeschnitten.

Im Unterschied zu der in den Figuren 1 - 7 beschriebenen Ausführung sind beim Zusatzmodul 102 gemäss den Figuren 8 und 9 die Haltearme, der Löseknopf 150 und die Halteeinrichtung in einem proximalen Bereich des Zusatzmoduls 102 angeordnet. Entsprechend wirken die Haltearme 143.1 nicht wie bei der Ausführung gemäss den Figuren 1 - 7 mit distalen Aussparungen im Injektor 101 zusammen, sondern mit Aussparungen, welche in Längsrichtung in einem mittleren Bereich des Injektors 101 angeordnet sind. Zudem sind die Haltearme 143.1 des Zusatzmoduls 102 gemäss den Figuren 8 und 9 anders ausgeformt. Ferner ist auch die Halteeinrichtung anders ausgebildet.

Wie bei der Ausführung in den Figuren 1 - 7 ist der erste der zwei Haltearme 143.1 auf einer ersten Seite und der zweite Haltearm auf einer der ersten Seite gegenüberliegenden Seiten der im Wesentlichen quadratischen Form der Aufnahmeeinheit 140 angeordnet. Allerdings haben die Haltearme 143.1 keine in radialer Richtung auf den Haltearmen angeordnete Auskragungen sondern in einer Richtung quer zur Längsachse des Zusatzmoduls 102 jeweils seitlich der Haltearme 143.1 angeordnete Führungsstifte 148.1, 148.2. Die Haltearme 143.1 weisen zudem wie in der ersten Ausführung beschrieben auf einer Innenseite, welche dem Innenraum der Aufnahmeeinheit 140 zugewandt ist, je einen Vorsprung auf (nicht ersichtlich in den Figuren 8 und 9). Diese Vorsprünge greifen in der Haltekonfiguration jeweils in Aussparungen im Injektor 101 ein.

Die Aufnahmeeinheit 140 weist je auf zwei gegenüberliegenden Seiten eine U-förmige Aussparung 190 auf mit einem ersten Schenkel 149.1 und einem zweiten Schenkel 149.2. Der Löseknopf 150 weist zwei einander gegenüberliegende in Richtung der Längsachse der Aufnahmeeinheit 140 ausgerichtete Schnapparme 157 auf, welche je an ihren freien Enden einen Nocken 158 umfassen. Dieser greift in der Haltekonfiguration in den ersten Schenkel 149.1 der U-förmigen Aussparung 142 ein, wodurch die Aufnahmeeinheit 140 verriegelt ist und nicht in distaler Richtung bewegt werden kann.

Um von Haltekonfiguration in die Freigabekonfiguration zu wechseln, wird der Löseknopf 150 in radiale Richtung zum Gehäuseeinsatz 30 hin gedrückt, wodurch die Schnapparme 157 relativ zum Gehäuseeinsatz 30 bewegt werden, so dass sie nach unten aus dem Schenkel 149.1 der U-förmigen Aussparung 142 hinausbewegt werden. Dadurch wird die Aufnahmeeinheit 140 freigegeben und kann sich in distaler Richtung verschieben, wodurch der Injektor 101 von der Aufnahmeeinheit 140 freigegeben wird.

Beim Loslassen des Löseknopfs 150 bewegt sich dieser wieder radial nach aussen. Dadurch werden die Nocken 158 der Schnapparme 157 in den zweiten Schenkel 149.2 der U-förmigen Aussparung 142 eingeführt.

Bei einer distalen Verschiebung der Aufnahmeeinheit 140 relativ zum Gehäuseeinsatz 130 werden die Führungsstifte 148.1, 148.2 der Haltearme 143.1 in seitliche Öffnungen 131.1 im Gehäuseeinsatz 130 eingeführt analog der Führung der Haltearme bei der ersten beschriebenen Ausführung gemäss den Figuren 1 - 7. Durch diese Bewegung werden die Haltearme 143.1 in den Öffnungen 131.1 schräg nach aussen vom Innenraum der Aufnahmeeinheit 140 wegbewegt. Dadurch wird der Injektor 101 von den Vorsprüngen der Haltearme 143.1 freigegeben.

Beim Verstellen des Zusatzmoduls von Freigabekonfiguration in die Haltekonfiguration wird die Aufnahmeeinheit in proximaler Richtung relativ zum Gehäuseeinsatz 130 verschoben. Dabei werden die Nocken 158 der Schnapparme 157 vom ersten Schenkel 149.1 der U-förmigen Aussparung 142 zum zweiten Schenkel 149.2 verschoben. Um dies zu ermöglichen weisen jeweils die distalen Seitenwände der Schenkel 149.1, 149.2 eine Anschrägung auf, auf denen die Nocken 158 aus den Schenkel 149.1 , 149.2 herausgleiten können.

In einer weiteren Ausführung können die Schnapparme auch um 180° gedreht angeordnet sein als in der Ausführung in den Figuren 8 und 9 gezeigt. In diesem Fall ist das freie Ende der Schnapparme zum proximalen Ende des Gehäuses 20 hin gewandt. Entsprechend weisen in dieser Ausführung die proximalen Seitenwände der Schenkel eine Anschrägung auf, so dass die Nocken der Schnapparme

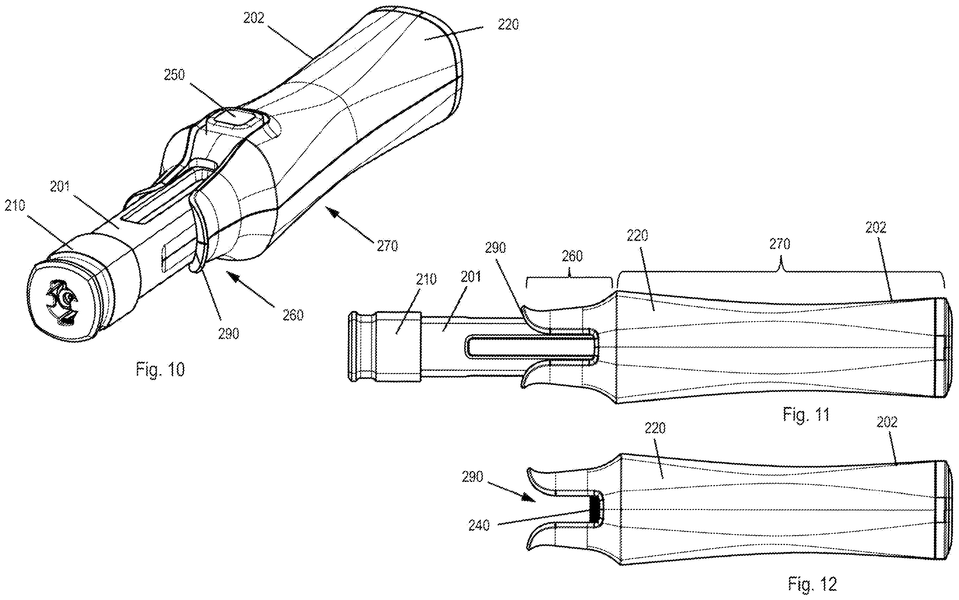

In den Figuren 10 - 12 ist eine weitere Ausführung des erfindungsgemässen Zusatzmoduls 202 dargestellt. Figur 10 zeigt eine perspektivische Ansicht von vorne während Figur 11 eine Rückansicht des Zusatzmoduls 202 zeigt. Figur 12 stellt eine Ansicht des Zusatzmoduls 202 ohne eingesetzten Injektor 201 dar.

Die in diesen Figuren gezeigte Ausführung hat im Unterschied zu den oben beschriebenen Ausführungen eine anders ausgeformte Aussenkontur. So ist in den Figuren 10 - 12 erkennbar, dass das Gehäuse 220 des Zusatzmoduls 202 einen distalen Abschnitt 260 und einen proximalen Abschnitt 270 aufweist. Der Umfang des distalen Abschnitts 260 nimmt zum proximalen Abschnitt 270 hin zu, wodurch eine Anschrägung gebildet wird.

Im Vergleich zum distalen Abschnitt 260 ist der proximale Abschnitt 270 wesentlich länger und weist eine konkave Form auf. Diese bildet eine Grifffläche, an welcher der Benutzer das Zusatzmodul 202 halten kann. Auch der distale Abschnitt 260 kann als Griff dienen, nämlich dann, wenn der Benutzer vor dem Gebrauch eine Nadelschutzkappe 210 in axialer Richtung vom Injektor 201 abziehen muss. Während der Benutzer das Zusatzmodul 202 mit einem oder mit mehreren Fingern am distalen Abschnitt 260 umgreift, dient die Anschrägung des distalen Abschnitts 260 als axiale Abstützung für die Hand, so dass die Nadelschutzkappe 210 problemlos vom Injektor 201 entfernt werden kann.

Die Halteeinrichtung mit dem Löseknopf 250 und den Haltearmen sind wie bei der Ausführung gemäss den Figuren 8 und 9 in einem distalen Bereich des Gehäuses 220 angeordnet. Im Unterschied zu den oben beschriebenen Ausführungen gemäss Figur 1 - 9 ist die Öffnung 290 im Gehäuse 220, in welche der Injektor 201 eingeführt wird, trichterförmig ausgebildet, wodurch sich der Injektor 201 einfacher einführen lässt. Die trichterförmige Öffnung 290 ist in Unfangrichtung unterbrochen, so dass die Sichtfenster des Injektors nicht durch das Gehäuse 220 des Zusatzmoduls 202 verdeckt werden.

Wenn der Injektor 201 nicht mit dem Zusatzmodul 202 verbunden ist, wie in Figur 12 gezeigt, steht die Aufnahmeeinheit etwas über eine distale Abschlusskante des Gehäuses 220 hinaus, so dass das ein schmaler Bereich 240 der Aufnahmeeinheit von aussen ersichtlich ist. Dieser Bereich 240 der Aufnahmeeinheit ist vorzugsweise rot markiert, damit er für den Benutzer gut ersichtlich ist. Wird der Injektor 201 in die Öffnung 290 eingeschoben und dadurch die Aufnahmeeinheit relativ zum Gehäuse 220 verschoben, wie oben beschrieben, verschwindet der Bereich 240 der Aufnahmeeinheit im Gehäuse 220. Wenn sich das Zusatzmodul 202 in seiner Haltekonfiguration befindet, ist der rot markierte Bereich 240 der Aufnahmeeinheit von aussen nicht mehr ersichtlich. Dadurch wird dem Benutzer angezeigt, dass der Injektor 201 korrekt mit dem Zusatzmodul 202 verbunden ist. Wenn hingegen die Haltearme nicht richtig in den Injektor 201 eingreifen und die Haltearme nicht durch den Gehäuseeinsatz verriegelt sind, bleibt zumindest ein Teil des rot markierten Bereichs 240 von aussen ersichtlich. Dadurch wird angezeigt, dass der Injektor 201 nicht korrekt mit dem Zusatzmodul 202 verbunden ist und sich das Zusatzmodul 202 folglich nicht in der Haltekonfiguration befindet.

Die Erfindung ist nicht auf die oben beschriebenen Ausführungsbeispiele beschränkt. So kann sich zum Beispiel in einer Alternative zu den Ausführungen gemäss den Figuren 1 - 12 die Lösestellung des Führungsnockens in einem unteren Bereich in der Nähe der Freigabestellung befindet. Das heisst, der Löseknopf verbleibt nach Betätigung in einer gedrückten Stellung und wird erst radial nach aussen verschoben, wenn ein Injektor korrekt eingesetzt ist und sich das Zusatzmodul in der Haltekonfiguration befindet.

Weiter kann die Verriegelung der Aufnahmeeinheit am Gehäuse anders als mit einem Führungsnocken und einem Sperrnocken realisiert sein. Beispielsweise kann die Aufnahmeeinheit mittels einer Gewindeverbindung am Gehäuse gesichert werden. Auch muss die Verstellung von der Haltekonfiguration in die Freigabekonfiguration nicht zwingend mit einem Löseknopf erfolgen. Vielmehr kann in einer Variante das Zusatzmodul auch durch manuelles Verschieben der Aufnahmeeinheit verstellt werden

Die Ausführung der Erfindung kann aber auch sonst von den beschriebenen Ausführungsbeispielen abweichen. So kann zum Beispiel das Zusatzmodul auch nur einen Haltearm und/oder nur einen Führungsnocken oder nur einen Sperrnocken aufweisen.

Weiter kann beispielsweise das Zusatzmodul auch für einen anderen Injektor als für einen Autoinjektor eingesetzt werden. Das Gehäuse des Zusatzmoduls kann eine andere Form als die in den Figuren 1 bis 12 gezeigt Form aufweisen. Insbesondere muss das Gehäuse den Injektor nicht vollständig umgeben, sondern kann zum Beispiel den Injektor nur über einen Abschnitt oder über einen Teilumfang aufnehmen. Zudem kann auch die Aufnahmeeinheit anders als hülsenförmig ausgeführt sein. Sie kann zum Beispiel viel kürzer, in der Form eines Rings oder einer Schelle, an dem die Halteelemente angeordnet sind, ausgebildet sein.

Ausserdem ist die Druckfeder zwischen dem Anschlagelement und der Gehäusewandung des Gehäuses nicht zwingend. Der erfindungsgemässe Mechanismus funktioniert auch ohne Druckfeder. Die Aufnahmeeinheit kann in diesem Fall von Hand verstellt werden. Ferner ist nicht zwingend ein Gehäuseeinsatz notwendig. Die Führung der Haltearme kann ebenso direkt im Gehäuse oder in einem anderen Element ausgebildet sein.

### BEZUGSZEICHENLISTE

- 1, 101, 201: Injektor
- 2, 102, 202: Zusatzmodul
- 11.1, 11.2: Aussparungen

- 20, 220: Gehäuse
- 21: obere Gehäusehälfte
- 22: untere Gehäusehälfte
- 23: Öffnung

- 30, 130: Gehäuseeinsatz
- 31.1,31.2: Öffnung
- 32.1,32.2: Auflageflächen
- 33: Durchbruch
- 34: Rippen

- 40, 140: Aufnahmeeinheit
- 41: Abschnitt
- 42: Sperrnocken
- 43.1., 43.2, 143.1: Haltearm
- 44.1, 44.2: Auskragung
- 45.1, 45.2: Vorsprung
- 46.1, 46.2: proximale Gleitfläche
- 47.1, 47.2: distale Gleitfläche
- 49: Kontaktfläche

- 50, 150, 250: Löseknopf
- 51: Löseknopffeder
- 53: Betätigungsfläche
- 54: Führungsnocken
- 55: Führungsfläche
- 56: Verriegelungsfläche

- 60: Elektronikmodul
- 70: Anschlagelement
- 71: Anschlagfläche
- 80: Druckfeder

- 142: U-förmige Aussparung
- 148.1, 148.2: Führungsstifte
- 149.1, 149.2: Schenkel
- 157: Schnapparm
- 158: Nocken

- 210: Nadelschutzkappe
- 240: Bereich Aufnahmeeinheit
- 260: distaler Abschnitt
- 270: proximaler Abschnitt
- 290: Öffnung

## Patentansprüche

1. Zusatzmodul (2) zum Verbinden mit einem Injektor (1) zur Abgabe einer medizinischen Substanz, umfassend ein Gehäuse (20) mit einer Halteeinrichtung und eine Aufnahmeeinheit (40) mit einem Halteelement (43.1, 43.2) zum Halten des Injektors (1) an oder in der Aufnahmeeinheit (40), wobei die Aufnahmeeinheit (40) relativ zum Gehäuse (20) bewegbar ist, wobei
a) das Zusatzmodul (2) eine Haltekonfiguration aufweist, in der die Aufnahmeeinheit (40) durch die Halteeinrichtung im Gehäuse (20) gehalten ist und nicht frei relativ zum Gehäuse (20) bewegbar ist und in der das Halteelement (43.1, 43.2) durch das Gehäuse verriegelt ist,
b) das Zusatzmodul (2) eine Freigabekonfiguration aufweist, in der die Aufnahmeeinheit (40) von der Halteeinrichtung freigegeben ist und so gelagert ist, dass sie frei relativ zum Gehäuse bewegbar ist und sich nicht vom Gehäuse lösen kann und in der das Halteelement (43.1, 43.2) von der Verriegelung gelöst ist
wobei das Zusatzmodul (2) durch eine Verstellbewegung der Aufnahmeeinheit (40) relativ zum Gehäuse von der Haltekonfiguration in die Freigabekonfiguration und zurück verstellbar ist.

2. Zusatzmodul (2) nach Anspruch 1, **dadurch gekennzeichnet, dass** die Verstellbewegung linear ist.

3. Zusatzmodul (2) nach einem der Ansprüche 1 bis 2, **dadurch gekennzeichnet, dass** das Gehäuse (20) hülsenförmig ausgebildet ist und in der Haltekonfiguration den in das Gehäuse (20) eingeführten Injektor (1) umgibt.

4. Zusatzmodul (2) nach einem der Ansprüche 1 bis 3, **dadurch gekennzeichnet, dass** die Aufnahmeeinheit (40) durch ein elastisches Element (80) in Richtung einer Längsachse des Gehäuses (20) in der Haltekonfiguration vorgespannt ist.

5. Zusatzmodul (2) nach einem der Ansprüche 1 bis 4, **dadurch gekennzeichnet, dass** das Halteelement (43.1, 43.2) zum Halten des Injektors (1) im Wesentlichen in eine Richtung senkrecht zur Längsachse des Zusatzmoduls bewegbar ist und dass das Halteelement (43.1, 43.2) einen Vorsprung (45.1. 45.2) zum Zusammenwirken mit dem Injektor (1) aufweist.

6. Zusatzmodul (2) nach Anspruch 5, **dadurch gekennzeichnet, dass** das Halteelement (43.1, 43.2) im Bereich eines freien Endes des Halteelements (43.1, 43.2) eine Auskragung (44.1, 44.2) zum Eingreifen in das Gehäuse (20) zwecks forcierter Lösung des Vorsprungs (45.1, 45.2) vom Injektor (1) umfasst.

7. Zusatzmodul (2) nach Anspruch 6, **dadurch gekennzeichnet, dass** sich in der Haltekonfiguration das Halteelement (43.1, 43.2) in einer Halteposition befindet, in der die Auskragung (44.1, 44.2) durch das Gehäuse (20) verriegelt ist, so dass das Halteelement (43.1, 43.2) nicht relativ zur Aufnahmeeinheit (40) bewegt werden kann und in der der Vorsprung in den im Gehäuse (20) eingesetzten Injektor (1) eingreift.

8. Zusatzmodul (2) nach Anspruch 6 oder 7, **dadurch gekennzeichnet, dass** sich in der Freigabekonfiguration das Halteelement (43.1, 43.2) in einer Freigabeposition befindet, in der die Auskragung (44.1, 44.2) in einer Ausnehmung im Gehäuse (20) aufgenommen ist und in der der Vorsprung (45.1, 45.2) von einem Innenraum der Aufnahmeeinheit (40) zurückgezogen ist, so dass der Injektor (1) vom Vorsprung (45.1, 45.2) freigegeben ist.

9. Zusatzmodul (2) nach Anspruch 7 oder 8, **dadurch gekennzeichnet, dass** das Zusatzmodul (2) nur in die Haltekonfiguration verstellbar ist, wenn das Halteelement (43.1, 43.2) in seine Halteposition bewegbar ist.

10. Zusatzmodul (2) nach einem der Ansprüche 1 bis 9, **dadurch gekennzeichnet, dass** die Halteeinrichtung einen Führungsnocken (54) und die Aufnahmeeinheit (40) einen Sperrnocken (42) umfasst, wobei in der Haltekonfiguration der Führungsnocken (54) derart mit dem Sperrnocken (42) zusammenwirkt, dass die Aufnahmeeinheit (40) unbeweglich im Gehäuse (20) gehalten ist.

11. Zusatzmodul (2) nach Anspruch 10, **dadurch gekennzeichnet, dass** der Führungsnocken (54) entlang eines Sicherungswegs verschiebbar ist und von einer Haltestellung, in welcher der Führungsnocken (54) mit dem Sperrnocken (42) zusammenwirkt, in eine Freigabestellung, in der der Sperrnocken (42) vom Führungsnocken (54) freigegeben ist, verstellbar ist und wobei der Führungsnocken (54) durch ein elastisches Sicherungselement in seine Haltestellung vorspannbar ist.

12. Zusatzmodul (2) nach einem der Ansprüche 1 bis 11, **dadurch gekennzeichnet, dass** die Halteeinrichtung einen Löseknopf (50) umfasst, mit dem das Zusatzmodul (2) von seiner Haltekonfiguration in seine Freigabekonfiguration verstellbar ist.

13. Anordnung umfassend einen Injektor (1) zur Abgabe einer medizinischen Substanz und ein Zusatzmodul (2) gemäß Anspruch 1 zum Verbinden mit dem Injektor (1), das Zusatzmodul (2) umfassend ein Gehäuse (20) mit einer Halteeinrichtung und eine Aufnahmeeinheit (40), welche ein Halteelement (43.1, 43.2) zum Halten des Injektors (1) an oder in der Aufnahmeeinheit (40) umfasst, wobei
a) das Zusatzmodul (2) eine Haltekonfiguration aufweist, in der die Aufnahmeeinheit (40) durch die Halteeinrichtung im Gehäuse (20) gehalten ist und nicht frei relativ zum Gehäuse (20) bewegbar ist und in der das Halteelement (43.1, 43.2) in eine Aussparung im Injektor eingreifen kann, um den Injektor (1) zu halten;
b) das Zusatzmodul (2) eine Freigabekonfiguration aufweist, in der die Aufnahmeeinheit (40) von der Halteeinrichtung freigegeben ist und so gelagert ist, dass sie frei relativ zum Gehäuse bewegbar ist und sich nicht vom Gehäuse lösen kann und der Injektor (1) vom Halteelement (43.1, 43.2) freigegeben ist,
wobei das Zusatzmodul (2) durch eine Verstellbewegung der Aufnahmeeinheit (40) relativ zum Gehäuse von der Haltekonfiguration in die Freigabekonfiguration und zurück verstellbar ist.

14. Verfahren zum Verbinden eines Injektors (1) mit einem Zusatzmodul (2) gemäß Anspruch 1, umfassend die Schritte
a. Bereitstellen eines Zusatzmoduls (2) mit einem Halteelement in einer Freigabeposition;
b. Einführen des Injektors (1) in das Zusatzmodul (2) mit einer Einführbewegung;
c. Verstellen, durch das Einführen, einer Aufnahmeeinheit (40) im Gehäuse (20) des Zusatzmoduls (2) relativ zum Gehäuse (20);
d. Bewegen, durch das Verstellen, eines Halteelements (43.1, 43.2) von der Freigabeposition zum Injektor (1) hin in eine Halteposition, so dass das Halteelement (43.1, 43.2) mit dem Injektor (1) zusammenwirkt und dadurch der Injektor (1) in der Aufnahmeeinheit (40) gehalten wird;
e. Verriegeln, durch das Verstellen, des Halteelements (43.1, 43.2) durch das Gehäuse (20);
f. Sichern der Aufnahmeeinheit (40) am Zusatzmodul (2) durch eine Halteeinrichtung.

15. Verfahren nach Anspruch 14 durchgeführt mit einem Zusatzmodul (2) nach einem der Ansprüche 1 - 12.

## Claims

1. An additional module (2) for connection to an injector (1) for dispensing a medical substance, comprising a housing (20) having a holding apparatus and a receiving unit (40) having a holding element (43.1, 43.2) for holding the injector (1) on or in the receiving unit (40), wherein the receiving unit (40) is movable relative to the housing (20), wherein
a) the additional module (2) has a holding configuration in which the holding unit (40) is held in the housing (20) by the holding apparatus and is not freely movable relative to the housing (20) and in which the holding element (43.1, 43.2) is locked by the housing,
b) the additional module (2) has a release configuration in which the holding unit (40) is released from the holding apparatus and is mounted in such a way that it can move freely relative to the housing and cannot detach from the housing and in which the holding element (43.1, 43.2) is released from the locking mechanism
wherein the additional module (2) can be adjusted from the holding configuration to the release configuration and back by an adjusting movement of the receiving unit (40) relative to the housing.

2. The additional module (2) according to claim 1, **characterized in that** the adjustment movement is linear.

3. The additional module (2) according to any of claims 1 to 2, **characterized in that** the housing (20) is designed in the shape of a sleeve and, in the holding configuration, surrounds the injector (1) inserted into the housing (20).

4. The additional module (2) according to any of claims 1 to 3, **characterized in that** the receiving unit (40) is biased by an elastic element (80) in the direction of a longitudinal axis of the housing (20) in the holding configuration.

5. The additional module (2) according to any of claims 1 to 4, **characterized in that** the holding element (43.1, 43.2) for holding the injector (1) is movable substantially in a direction perpendicular to the longitudinal axis of the additional module, and **in that** the holding element (43.1, 43.2) has a protrusion (45.1. 45.2) for cooperating with the injector (1).

6. The additional module (2) according to claim 5, **characterized in that** the holding element (43.1, 43.2) comprises a projection (44.1, 44.2) in the area of a free end of the holding element (43.1, 43.2) for engaging in the housing (20) for the purpose of forced release of the protrusion (45.1, 45.2) from the injector (1).

7. The additional module (2) according to claim 6, **characterized in that** in the holding configuration, the holding element (43.1, 43.2) is located in a holding position in which the protrusion (44.1, 44.2) is locked by the housing (20), so that the holding element (43.1, 43.2) cannot be moved relative to the receiving unit (40) and in which the protrusion engages in the injector (1) inserted in the housing (20).

8. The additional module (2) according to claim 6 or 7, **characterized in that** in the release configuration, the holding element (43.1, 43.2) is in a release position in which the protrusion (44.1, 44.2) is received in a recess in the housing (20) and in which the protrusion (45.1, 45.2) is retracted from an interior space of the receiving unit (40), so that the injector (1) is released from the protrusion (45.1, 45.2).

9. The additional module (2) according to claim 7 or 8, **characterized in that** the additional module (2) is only adjustable into the holding configuration when the holding element (43.1, 43.2) is movable into its holding position.

10. The additional module (2) according to any of claims 1 to 9, **characterized in that** the holding apparatus comprises a guide cam (54) and the receiving unit (40) comprises a locking cam (42), wherein in the holding configuration the guide cam (54) cooperates with the locking cam (42) in such a manner that the receiving unit (40) is held immovably in the housing (20).

11. The additional module (2) according to claim 10, **characterized in that** the guide cam (54) is displaceable along a securing path and is adjustable from a holding position, in which the guide cam (54) cooperates with the locking cam (42), into a release position, in which the locking cam (42) is released from the guide cam (54), and wherein the guide cam (54) can be biased into its holding position by an elastic securing element.

12. The additional module (2) according to any of claims 1 to 11, **characterized in that** the holding apparatus comprises a release button (50) with which the additional module (2) can be adjusted from its holding configuration to its release configuration.

13. An arrangement comprising an injector (1) for dispensing a medical substance and an additional module (2) according to claim 1 for connection to the injector (1), the additional module (2) comprising a housing (20) having a holding apparatus and a receiving unit (40) which comprises a holding element (43.1, 43.2) for holding the injector (1) on or in the receiving unit (40), wherein
a) the additional module (2) has a holding configuration in which the holding unit (40) is held in the housing (20) by the holding apparatus and is not freely movable relative to the housing (20) and in which the holding element (43.1, 43.2) can engage in a recess in the injector in order to hold the injector (1);
b) the additional module (2) has a release configuration in which the receiving unit (40) is released from the holding apparatus and is mounted in such a manner that it can move freely relative to the housing and cannot detach from the housing and the injector (1) is released from the holding element (43.1, 43.2),
wherein the additional module (2) can be adjusted from the holding configuration to the release configuration and back by an adjusting movement of the receiving unit (40) relative to the housing.

14. A method for connecting an injector (1) to an additional module (2) according to claim 1, comprising the steps of
a. providing an additional module (2) with a holding element in a release position;
b. inserting the injector (1) into the additional module (2) with an insertion movement;
c. adjusting, by inserting, a receiving unit (40) in the housing (20) of the additional module (2) relative to the housing (20);
d. moving, by adjusting, a holding element (43.1, 43.2) from the release position towards the injector (1) into a holding position, in such a manner that the holding element (43.1, 43.2) interacts with the injector (1) and the injector (1) is thereby held in the receiving unit (40);
e. locking, by moving the holding element (43.1, 43.2) through the housing (20);
f. securing the mounting unit (40) to the additional module (2) by means of a holding apparatus.

15. The method according to claim 14 performed with an additional module (2) according to any of claims 1 to 12.

## Revendications

1. Module supplémentaire (2) destiné à être relié à un injecteur (1) pour la distribution d'une substance médicale, comprenant un boîtier (20) comportant un dispositif de maintien et une unité de réception (40) comportant un élément de maintien (43.1, 43.2) permettant le maintien de l'injecteur (1) sur ou dans l'unité de réception (40), dans lequel l'unité de réception (40) est mobile par rapport au boîtier (20), dans lequel
a) le module supplémentaire (2) présente une configuration de maintien, dans laquelle l'unité de réception (40) est maintenue dans le boîtier (20) par le dispositif de maintien et n'est pas mobile librement par rapport au boîtier (20) et dans laquelle l'élément de maintien (43.1, 43.2) est verrouillé par le boîtier,
b) le module supplémentaire (2) présente une configuration de libération, dans laquelle l'unité de réception (40) est libérée du dispositif de maintien et est montée de sorte qu'elle est mobile librement par rapport au boîtier et ne peut pas se détacher du boîtier et dans laquelle l'élément de maintien (43.1, 43.2) est détaché du verrouillage
dans lequel le module supplémentaire (2) peut être déplacé de la configuration de maintien à la configuration de libération et inversement par un mouvement de déplacement de l'unité de réception (40) par rapport au boîtier.

2. Module supplémentaire (2) selon la revendication 1,
**caractérisé en ce que** le mouvement de déplacement est linéaire.

3. Module supplémentaire (2) selon l'une des revendications 1 à 2,
**caractérisé en ce que** le boîtier (20) est réalisé en forme de manchon et entoure, dans la configuration de maintien, l'injecteur (1) introduit dans le boîtier (20).

4. Module supplémentaire (2) selon l'une des revendications 1 à 3,
**caractérisé en ce que** l'unité de réception (40) est précontrainte par un élément élastique (80) en direction d'un axe longitudinal du boîtier (20) dans la configuration de maintien.

5. Module supplémentaire (2) selon l'une des revendications 1 à 4,
**caractérisé en ce que** l'élément de maintien (43.1, 43.2) permettant de maintenir l'injecteur (1) est mobile sensiblement dans une direction perpendiculaire à l'axe longitudinal du module supplémentaire **et en ce que** l'élément de maintien (43.1, 43.2) présente une saillie (45.1. 45.2) permettant de coopérer avec l'injecteur (1).

6. Module supplémentaire (2) selon la revendication 5,
**caractérisé en ce que** l'élément de maintien (43.1, 43.2) comprend, dans la zone d'une extrémité libre de l'élément de maintien (43.1, 43.2), un renflement (44.1, 44.2) permettant la mise en prise dans le boîtier (20) en vue d'un détachement forcé de la saillie (45.1, 45.2) de l'injecteur (1).

7. Module supplémentaire (2) selon la revendication 6,
**caractérisé en ce que**, dans la configuration de maintien, l'élément de maintien (43.1, 43.2) se trouve dans une position de maintien, dans laquelle le renflement (44.1, 44.2) est verrouillé par le boîtier (20), de sorte que l'élément de maintien (43.1, 43.2) ne peut pas être mu par rapport à l'unité de réception (40) et dans laquelle la saillie vient en prise dans l'injecteur (1) inséré dans le boîtier (20).

8. Module supplémentaire (2) selon la revendication 6 ou 7,
**caractérisé en ce que**, dans la configuration de libération, l'élément de maintien (43.1, 43.2) se trouve dans une position de libération, dans laquelle le renflement (44.1, 44.2) est reçu dans un évidement dans le boîtier (20) et dans laquelle la saillie (45.1, 45.2) est retirée d'un espace intérieur de l'unité de réception (40), de sorte que l'injecteur (1) est libéré de la saillie (45.1, 45.2).

9. Module supplémentaire (2) selon la revendication 7 ou 8,
**caractérisé en ce que** le module supplémentaire (2) ne peut être déplacé dans la configuration de maintien que lorsque l'élément de maintien (43.1, 43.2) est mobile dans sa position de maintien.

10. Module supplémentaire (2) selon l'une des revendications 1 à 9,
**caractérisé en ce que** le dispositif de maintien comprend une came de guidage (54) et l'unité de réception (40) comprend une came de blocage (42), dans lequel, dans la configuration de maintien, la came de guidage (54) coopère avec la came de blocage (42) de telle sorte que l'unité de réception (40) est maintenue de manière à être immobile dans le boîtier (20).

11. Module supplémentaire (2) selon la revendication 10,
**caractérisé en ce que** la came de guidage (54) peut coulisser le long d'un chemin de fixation et peut être déplacée d'une position de maintien, dans laquelle la came de guidage (54) coopère avec la came de blocage (42), à une position de libération, dans laquelle la came de blocage (42) est libérée de la came de guidage (54), et dans lequel la came de guidage (54) peut être précontrainte par un élément de fixation élastique dans sa position de maintien.

12. Module supplémentaire (2) selon l'une des revendications 1 à 11,
**caractérisé en ce que** le dispositif de maintien comprend un bouton de détachement (50) avec lequel le module supplémentaire (2) peut être déplacé de sa configuration de maintien à sa configuration de libération.

13. Agencement comprenant un injecteur (1) permettant la distribution d'une substance médicale et un module supplémentaire (2) selon la revendication 1 destiné à être relié à l'injecteur (1), le module supplémentaire (2) comprenant un boîtier (20) comportant un dispositif de maintien et une unité de réception (40), laquelle comprend un élément de maintien (43.1, 43.2) permettant le maintien de l'injecteur (1) sur ou dans l'unité de réception (40), dans lequel
a) le module supplémentaire (2) présente une configuration de maintien, dans laquelle l'unité de réception (40) est maintenue dans le boîtier (20) par le dispositif de maintien et n'est pas mobile librement par rapport au boîtier (20) et dans laquelle l'élément de maintien (43.1, 43.2) peut venir en prise dans une cavité dans l'injecteur afin de maintenir l'injecteur (1) ;
b) le module supplémentaire (2) présente une configuration de libération, dans laquelle l'unité de réception (40) est libérée du dispositif de maintien et est montée de sorte qu'elle est mobile librement par rapport au boîtier et ne peut pas se détacher du boîtier et l'injecteur (1) est libéré de l'élément de maintien (43.1, 43.2), dans lequel le module supplémentaire (2) peut être déplacé de la configuration de maintien à la configuration de libération et inversement par un mouvement de déplacement de l'unité de réception (40) par rapport au boîtier.

14. Procédé permettant de relier un injecteur (1) à un module supplémentaire (2) selon la revendication 1, comprenant les étapes consistant à
a. fournir un module supplémentaire (2) comportant un élément de maintien dans une position de libération ;
b. introduire l'injecteur (1) dans le module supplémentaire (2) avec un mouvement d'introduction ;
c. déplacer, par l'introduction, une unité de réception (40) dans le boîtier (20) du module supplémentaire (2) par rapport au boîtier (20) ;
d. mouvoir, par le déplacement, un élément de maintien (43.1, 43.2) de la position de libération à une position de maintien vers l'injecteur (1), de sorte que l'élément de maintien (43.1, 43.2) coopère avec l'injecteur (1) et que l'injecteur (1) est ainsi maintenu dans l'unité de réception (40) ;
e. verrouiller, par le déplacement, l'élément de maintien (43.1, 43.2) par le boîtier (20) ;
f. fixer l'unité de réception (40) au module supplémentaire (2) par un dispositif de maintien.

15. Procédé selon la revendication 14 mis en oeuvre avec un module supplémentaire (2) selon l'une des revendications 1 à 12.
